(19) Europäisches Patentamt
European Patent Office
Office européen des brevets

(11) Publication number: **0 539 938 A1**

(12) # EUROPEAN PATENT APPLICATION

(21) Application number: **92118413.1**

(22) Date of filing: **28.10.92**

(51) Int. Cl.5: **C07D 405/06**, C07D 249/12, C07D 295/08, C07D 207/09, C07D 405/14, A61K 31/41

(30) Priority: **30.10.91 US 785357**
**01.07.92 US 907262**

(43) Date of publication of application:
**05.05.93 Bulletin 93/18**

(84) Designated Contracting States:
**PT**

(71) Applicant: **SCHERING CORPORATION**
**2000 Galloping Hill Road**
**Kenilworth New Jersey 07033(US)**

(72) Inventor: **Saksena, Anil K.**
**53 Beverly Road**
**Upper Montclair, New Jersey 07043(US)**
Inventor: **Girijavallabhan, Viyyoor M.**
**10 Maplewood Drive**
**Parsippany, New Jersey 07054(US)**
Inventor: **Ganguly, Ashit K.**
**96 Cooper Avenue**
**Upper Montclair, New Jersey 07043(US)**
Inventor: **Lovey, Raymond G.**
**65 Woodside Avenue**
**West Caldwell, New Jersey 07006(US)**

(74) Representative: **von Kreisler, Alek, Dipl.-Chem. et al**
**Patentanwälte Von Kreisler-Selting-Werner, Deichmannhaus am Hauptbahnhof**
**W-5000 Köln 1 (DE)**

(54) **Tri-substituted tetrahydrofuran antifungals.**

(57) An antifungal compound represented by formula [I]:

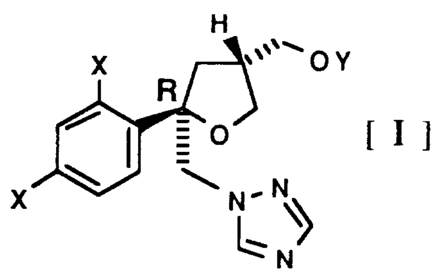

[ I ]

wherein X is independently both F or both Cl or one X is F and the other is Cl;
Y =



Rank Xerox (UK) Business Services
(3.10/3.6/3.3.1)

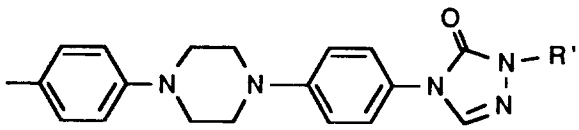 ; 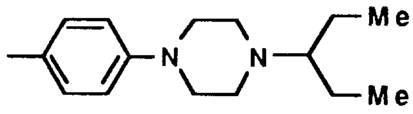 ;

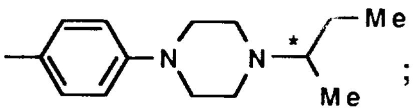 ; 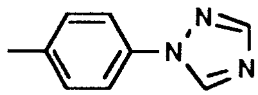 ;

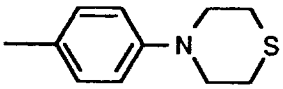 ; 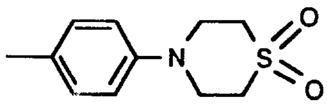 ; or

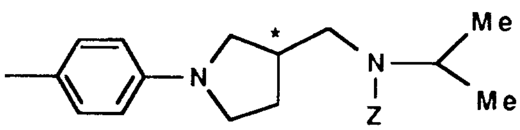

R' = $(C_1-C_{10})$ alkyl; $(C_2-C_{10})$alkenyl; $(C_2-C_{10})$alkynyl; $(C_3-C_8)$cycloalky or $CH_2R^2$;
$R^2$ = $(C_1-C_3)$perhaloalkyl; $CO_2R^3$ *$CH(OR^4)CH_2OR^4$ or $CH_2N(R^5)$
$R^3$ = lower alkyl or H
$R^4$ = $R^3$ or $(CH_2)_2OR^3$
$R^5$ = lower alkyl

Z = H, or $(C_1-C_5)$ alkanoyl and the carbons with the asterisks (*) have the R or S absolute configuration; or a pharmaceutically acceptable salt thereof as well as pharmacetical compositions containing them and a methods of treating or preventing fungal infections in mammals using them are disclosed.

## BACKGROUND OF THE INVENTION

This invention relates to tri-substituted tetrahydrofuran antifungals, such as (-)-[(5R)-cis-[-4-[4-[4-[4-[[5-(2,4-dihalophenyl)-5-(1H-1,2,4-triazol-1-ylmethyl)tetrahydrofuran-3-yl]-methoxy]phenyl] substituted antifungals, pharmaceutical compositions containing them, tri-substituted tetrahydrofuran antifungal intermediates, and methods of treating and/or preventing antifungal infections in hosts, including warm-blooded animals, especially humans with such tri-substituted tetrahydrofuran antifungals.

International Publication Number WO 89/04829, published 1 June 1990 and USP 5,039,676 (A.K. Saksena et al.) discloses (±) cis and (±) (trans antifungal compounds represented by the formula

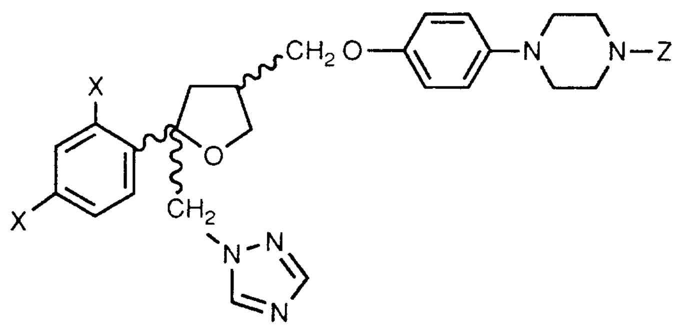

wherein X= F, Cl; Z=loweralkyl, ($C_2-C_8$) alkanoyl or phenyl substituted by 2-loweralkyl-3-oxo-1,2,4-triazol-4-yl,e.g., (±)-cis and (±)-trans-1-[4-[[2-(2,4-difluorophenyl)-2-[(1H-1,2,4-triazol-1-yl)methyl]tetrahydro-4-furanyl]methoxy]phenyl]-4-(1-methylethyl)piperazine. However, WO 89/04829 does not specifically disclose the compounds of this invention.

There is a need for broad-spectrum antifungal agents to treat systemic fungal infections, especially Aspergillus and Candida infections.

## SUMMARY OF INVENTION

The present invention provides compounds represented by formula 1

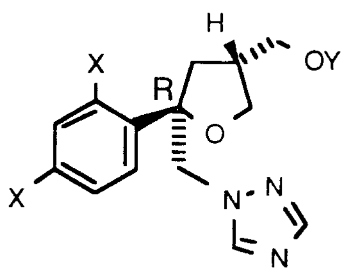

[ I ]

wherein X is independently both F or both Cl or one X is independently F and the other is Cl;
Y =

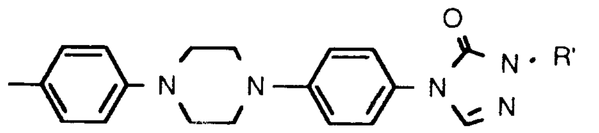;

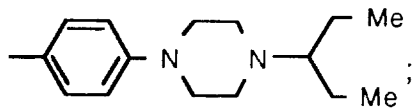;

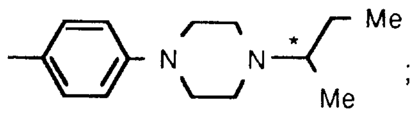; 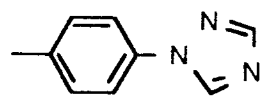;

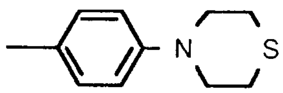; 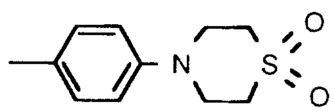; or

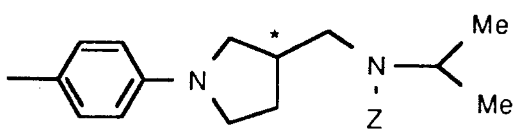

R' = $(C_1-C_{10})$alkyl; $(C_2-C_{10})$alkenyl; $(C_2-C_{10})$alkynyl; $(C_3-C_8)$cycloalkyl; or $CH_2R^2$;
$R^2$ = $(C_1-C_3)$ perhaloalkyl; $CO_2R^3$; *$CH(OR^4)CH_2OR^4$ or $CH_2N(R^5)_2$
$R^3$ = lower alkyl or H
$R^4$ = $R^3$ or $(CH_2)_2OR^3$
$R^5$ = lower alkyl

Z = H, or $(C_1-C_5)$ alkanoyl and the carbons with the asterisk (*) have the R or S absolute configuration; or a pharmaceutically acceptable salt thereof.

In a preferred aspect of the present invention there is provided compounds represented by formula Ia

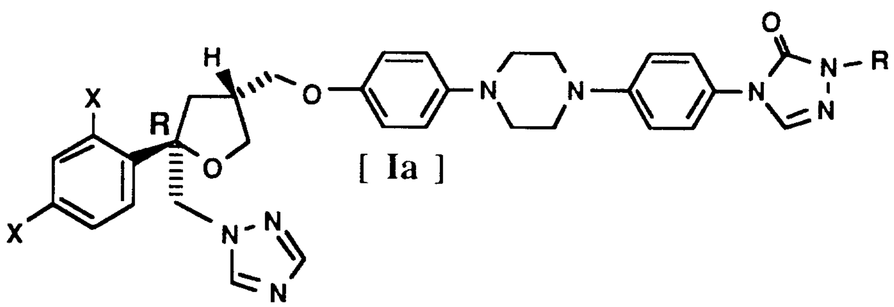

[ Ia ]

wherein X is independently both F or both Cl or one X is independently F and the other is independently Cl;

R' =

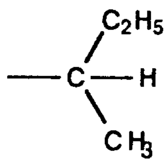 , 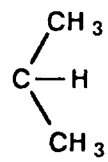 , 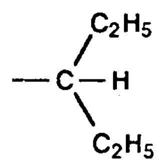 ,

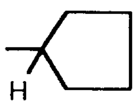 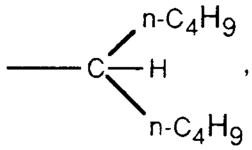 , 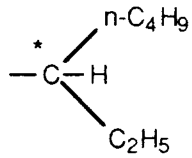 , $-CH_2CF_3$,

$-CH_2CO_2H$, $-CH_2CH_2N(CH_3)_2$, $-(CH_2)_4C\equiv CH$

$-CH_2CH=CHC_2H_5$, 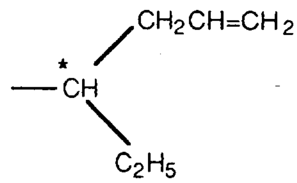 ,

$-CH_2-C\equiv C-CH_3$, 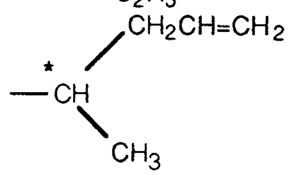 ,

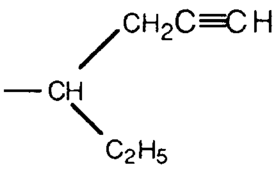 , 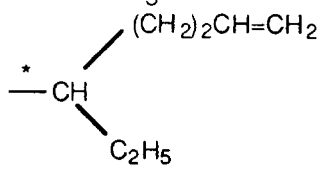 ,

$-(CH_2)CH=C(CH_3)_2$ $-CH_2CH=CHCH(CH_3)_3$ and

$-CH_2-{}^*CH(OH)CH_2OH$

and the carbons with the asterisk (*) have the R or S absolute configuration; or a pharmaceutically acceptable salt thereof.

The present invention also provides intermediates useful for the production of antifungal compounds represented by formula I. Thus, the present invention provides a compound represented by formula III – VII:

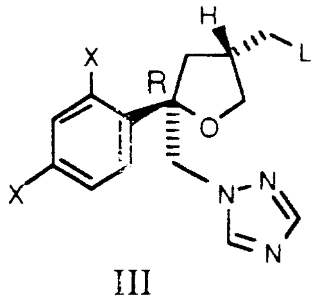

III

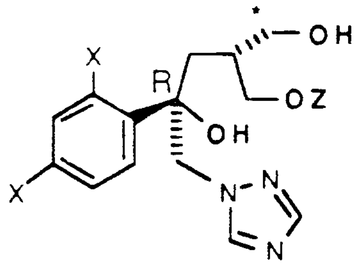

VII

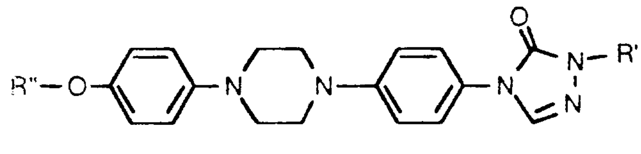

IV

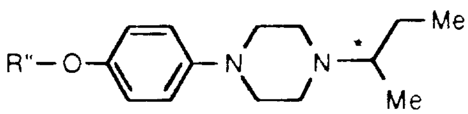

V

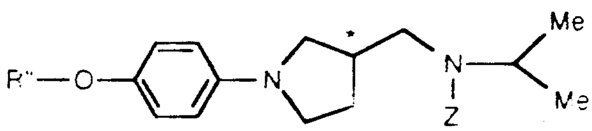

VI

wherein X is independently both F or both Cl or one X is independently F and the other is independently Cl;

R' =

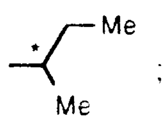 ; 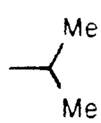 ; 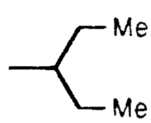 ;

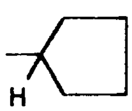 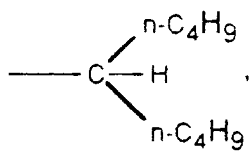 , 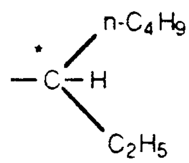 , $—CH_2CF_3$ ,

$—CH_2CO_2H$, $-CH_2CH_2N(CH_3)_2$, $-(CH_2)_4C\equiv CH$

$-CH_2CH=CHC_2H_5$, 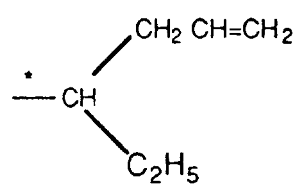 ,

$-CH_2-C\equiv C-CH_3$,

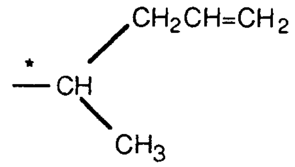,

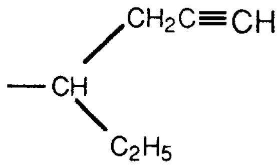,

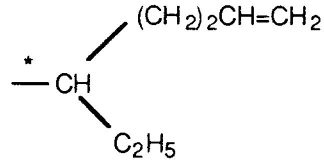,

$-(CH_2)CH=C(CH_3)_2$

$-CH_2-CH=CHCH(CH_3)_2$, and

$-CH_2-{}^*CH(OH)CH_2OH$

L is OH or LG; LG is a leaving group;
R'' is lower alkyl or Z and
Z = H or ($C_1-C_5$) alkanoyl and the carbons with the asterisks (*) have the R or S absolute configuration.

The carbon with the asterisk in the compound of formula VII may be in the R or S absolute configuration when Z is not equal to H; each optical isomer of VIII may be independently converted into the compounds of formula III by the synthetic steps of Scheme III listed hereinafter.

## BRIEF DESCRIPTION OF THE FIGURE

The sole figure illustrates the efficacy (PO) of preferred antifungal compounds of this invention, e.g., the compounds of formula IIa and IIb of this invention vs itraconazole, fluconazole and saperconazole in compromised mice infected by inhalation of *Aspergillus flavus* spores.

## DETAILED DESCRIPTION OF THE INVENTION AND OF THE PREFERRED EMBODIMENTS

The term "lower alkyl", as used herein, means straight and branched chain hydrocarbon groups of 1 to 6 carbon atoms, such as methyl, ethyl, *n*-, and *iso*-propyl, *n*-, *sec*- and *tert*-butyl, *n*-, *sec*-, *iso*-, *tert*- and *neo*-pentyl, *n*-, *sec*-, *iso*-, *tert*- and *neo*-hexyl and the like.

The term "($C_1-C_{10}$) alkyl", as used herein means straight and branched chain alkyl groups of one to ten carbons including but not limited to methyl, ethyl, *n* and *iso* propyl, *n*, *sec*, *iso* and *tert*-butyl, *n*-, *sec*-, *iso*-, *tert* and *neo*-pentyl *n*-, *sec*-, *iso*-, *tert*- and *neo*-hexyl, *n*-, *sec*-, *iso*-, *tert*- and *neo*-heptyl, *n*, *sec*- *iso*, *tert*-and *neo*-octyl, *n*, *sec*, *iso* *neo*- and *tert*-nonyl, and *n*, *sec*, *iso*, *tert*- *neo*-decyl.

The term "($C_2-C_{10}$) alkenyl, as used herein means straight and branched chain alkenyl groups of two to ten carbons containing at least one double bond, and including $-CH_2CH=CH_2$, $-CH_2CH=CH-CH_3$, $-(CH_2)_3CH=CHCH_3$, $-(CH_2)_2CH=CHCH_3$, $-CH_2CH=CHC_2H_5$, $-CH=CHCH(CH_3)_2$; $^*CH(CH_3)-CH_2CH=CH_2$, $-^*CH(C_2H_5)CH_2CH=CH_2$, $-^*CH(C_2H_5)(CH_2)_2CH=CH_2$, $-^*CH(C_3H_7)CH_2CH=CH_2$, $-^*CH-(C_4H_7)CH_2CH=CH_2$, $-^*CH(CH_3)CH_2CH=C(CH_3)_2$, $-^*CH(C_2H_3)CH_2CH=C(CH_3)_2$. The double bond may be in the *cis* or *trans* form; use of the *trans* isomer is preferred.

The term "($C_3-C_{10}$) alkynyl", as used herein means straight and branched chain alkyl groups of two to ten carbons containing at least one triple bond and including $-CH_2C\equiv CH_3$, $-CH\equiv C_2CH_5$, $-(CH_2)_3C\equiv CH$, $-C(CH_2)_4C\equiv CH$, $-(CH_2)_3C\equiv CCH_3$, $^*CH(CH_3)CH_2C\equiv CH$, $-^*CH(C_2H_5)CH_2C\equiv CCH$, $-^*CH(C_3H_7)(CH_2)-{}_2C\equiv CH_3$, $-^*CH(C_4H_9)(CH_2)_2C\equiv -CCH$, $-CH_2-C\equiv C-C\equiv C-C(CH_3)_3$ and $-CH_2-CH=CH-C\equiv C-C-(CH_3)_3$

The term "($C_3-C_8$) cycloalkyl", as used herein means cycloalkyl groups of three to eight carbons including, cyclopropyl -methylcyclopropyl, dimethylcyclopropyl, cyclobutyl, cyclopentyl, methylcyclopentyl, cyclohexyl, cycloheptyl and cyclooctyl.

The term "($C_1-C_3$) perhaloalkyl" as used herein means alkyl groups of one to three carbons wherein all the hydrogens are replaced by halogen, especially fluorine or chlorine. Typically suitable ($C_1-C_3$) perhaloalkyl include $CF_3-$, $CF_3CF_2-$, $CCl_3CCl_2-$ and *n* and *iso*-$C_3F_7$.

The term "leaving group" (LG) as used herein, means leaving groups readily displaceable with appropriate reactants under conventional conditions well known to those skilled in the organic synthetic arts

so as to form the compound represented by formula I. Typical suitable leaving groups include but are not limited to halide especially bromide but also iodide, trifluoromethylsulfonyloxy, methylsulfonyloxy, and 4-methyl-phenylsulfonyloxy.

The term "($C_1-C_5$) alkanoyl", as used herein means straight and branched chain alkanoyl groups of 1 to 5 carbon atoms such as formyl, acetyl, n- and iso-propionyl, n, sec-, and iso -butyryl and n-, sec, iso, and tert-pentanoyl.

The dihalophenyl group in the compounds of the invention includes 2,4-difluorophenyl; 2,4-dichlorophenyl; 2-chloro-4-fluorophenyl and 2-fluoro-4-chlorophenyl.

The compounds of the invention exhibit broad spectrum antifungal activity in various in vitro assays against yeasts, dematophytes and Aspergillus as well as in the following in vivo models: an Aspergillus pulmonary mouse model (PO and parenteral), a Candida systemic model (with normal and compromised mice, PO and parenteral), and in a Candida hamster vaginal model (PO and topically). For example, the preferred antifungal compounds represented by formulas IIa, IIb and IIc are more active orally against Aspergillus flavus pulmonary infections in an in vivo mouse model than itraconazole, fluconazole and saperconazole (See Comparative Example 31 and Table I and Figure 1). Compounds represented by formulas IIa, IIb and IIc were more active than itraconazole and saperconazole against (a) systemic candidiasis in normal and compromised mice (See Comparative Example 33 and Table II and comparative Example 36 and Table V) as well as in (b) a Candida vaginal infection in a hamster model.

The antifungal compounds of this invention represented by formula I have the R absolute stereochemical configuration at the carbon in the tetrahydrofuran ring bearing the di-halophenyl and 1H, 1,2,4-triazol-1-ylmethyl moieties, and the $CH_2OY$ moiety has the "cis" stereochemical configuration relative to the 1H, 1,2,4-triazol-1-ylmethyl moiety. See the formula I hereinbelow.

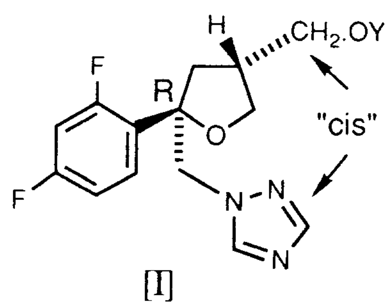


[I]

The compounds of formula I are generically but not specifically disclosed as the "cis" series, type ii, at col. 9 lines 59-68 of Saksena et al. USP 5,039,676 and Example 68 at Co. 5, line 16 to col. 52, line 44. The antifungal compounds of this invention e.g. of formula IIb exhibit oral activity in the Aspergillus pulmonary mouse model; the compound of Example 68 of US Patent 5,039,676 is inactive in this in vivo Aspergillus model. See Comparative Example 34 and Table III.

## GENERAL SYNTHETIC PREPARATIONS

The compounds of this invention may be prepared by use of the sequence of steps illustrated in the following Schemes I-III. In Scheme I, compound 3 is readily prepared from commercially available compound 1 according to examples 1a, 1b and 1c. Compound 4 is prepared by reaction of L(+) -diethyl tartarate ("L-DET") and molecular sieves in the presence of titanium tetra-isopropoxide ($(i-PrO)_4T_i$) in an aprotic solvent, such as methylene chloride, at a temperature 0° to -35°C. See for example - T. Katsuki, K.B. Sharpless, J. Am. Chem. Soc., 102, 5974 (1980); and 103, 464 (1981). An oxidizing agent, e.g. tert-butylhydroperoxide ("TBHP") is added to this reaction mixture (step d of Scheme I). Compound 3 is added and the compound of formula 4 (when L(+)-diethyl tartarate is used) is produced. Reaction of compound 4 with 1H-1,2,4-triazole in the presence of strong base, e.g., NaH in an aprotic solvent, such as DMF, at 0° -5°C provides the diol compound of formula 5. The primary hydroxy group in compound 5 is converted into a leaving group, e.g., mesylate or tosylate (compound 6) by reaction of 5 with, for example, mesyl chloride ("MsCl") in an aprotic solvent, e.g., methylene chloride in the presence of base, e.g., triethylamine ("$Et_3N$"). Compound 6 is treated with strong base, e.g., sodium hydride (NaH) in an aprotic solvent, e.g., DMF at room temperature to give oxirane compound 7. Reaction of 7 with diethyl malonate in the presence of strong base, e.g., sodium hydride in an aprotic solvent, e.g., DMSO at 25° -75°C provides the lactone

8. Reduction of 8 with a metal hydride, e.g., lithium borohydride ($LiBH_4$) in an alcohol, e.g., ethanol (EtOH), provides the triol 9. Conversion of the two primary alcohols of 9 into leaving groups (mesylates or tosylates) by reaction of 9 with excess tosyl chloride in an aprotic solvent, e.g., THF, in the presence of base, e.g., $Et_3N$, provides ditosylate 10. Compound 10 is contacted with strong base, e.g., NaH, in an aprotic solvent such as toluene at elevated temperatures of 100° – 120° C to provide a mixture of two tosylates (cis and trans) which are separated by chromatography to yield to the cis – tosylate 11. Reaction of compound 11 with alcohols HOY in the presence of strong base, such as NaH in an aprotic solvent, such as DMSO at a temperature of 25° – 75° C provides compounds of formula I.

Scheme II provides an alternative reaction sequence to obtain compounds of the present invention. Reaction of compound 11 with the commercially available compound 12 in the presence of NaH gives compound 13. Hydrolysis of N – acetyl group in 13 is accomplished with a strong base such as NaOH in the presence of n – BuOH to provide compound 14. It should be made clear that instead of N – acetyl group in compound 12, any other base labile groups such as N – formyl, N – benzoyl, etc., can also be used to provide corresponding N – formyl and N – benzoyl derivatives of compound 13. Reaction of 13 with p – chloronitrobenzene in the presence of a hydrochloric acid scavenger such as $K_2CO_3$ provides the nitro compound 15. Catalytic reduction of 15 in the presence of a platinum or palladium catalyst yields the amine 16. Treatment of 16 with phenylchloroformate in the presence of pyridine gives the urethane intermediate 17. Reaction of 17 with hydrazine yields the semicarbazide 18 which is cyclized in the presence of formamidine acetate to furnish the key triazolone 19. Alkylation of 19 according to Examples 19 and 20 provides the compounds of structure 20 including compounds of formulas IIa and IIb.

Scheme III provides a stereospecific access to the cis – alcohol 26 and cis – tosylate 11 by application of enzyme chemistry. For example, reaction of the triol 9 with ethyl acetate in the presence of porcine pancreatic lipase gives a single monoacetate 21. The remaining primary hydroxy group in 21 is protected by an acid labile group such as tetrahydropyranyl group to give a compound such as 22. Hydrolysis of the acetoxy group in 22 is accomplished with a base such a KOH which provides 23. The remaining steps are: (i) tosylation of compound 23 to provide 24; (ii) cyclization of 24 in the presence of NaH to provide 25 (iii) deprotection of THP ether in 25 using an acid catalyst such as p – toluene sulfonic acid (to give 26) followed by tosylation of the resulting 26 to furnish the key intermediate 11. (Examples 37 – 41)

Scheme I

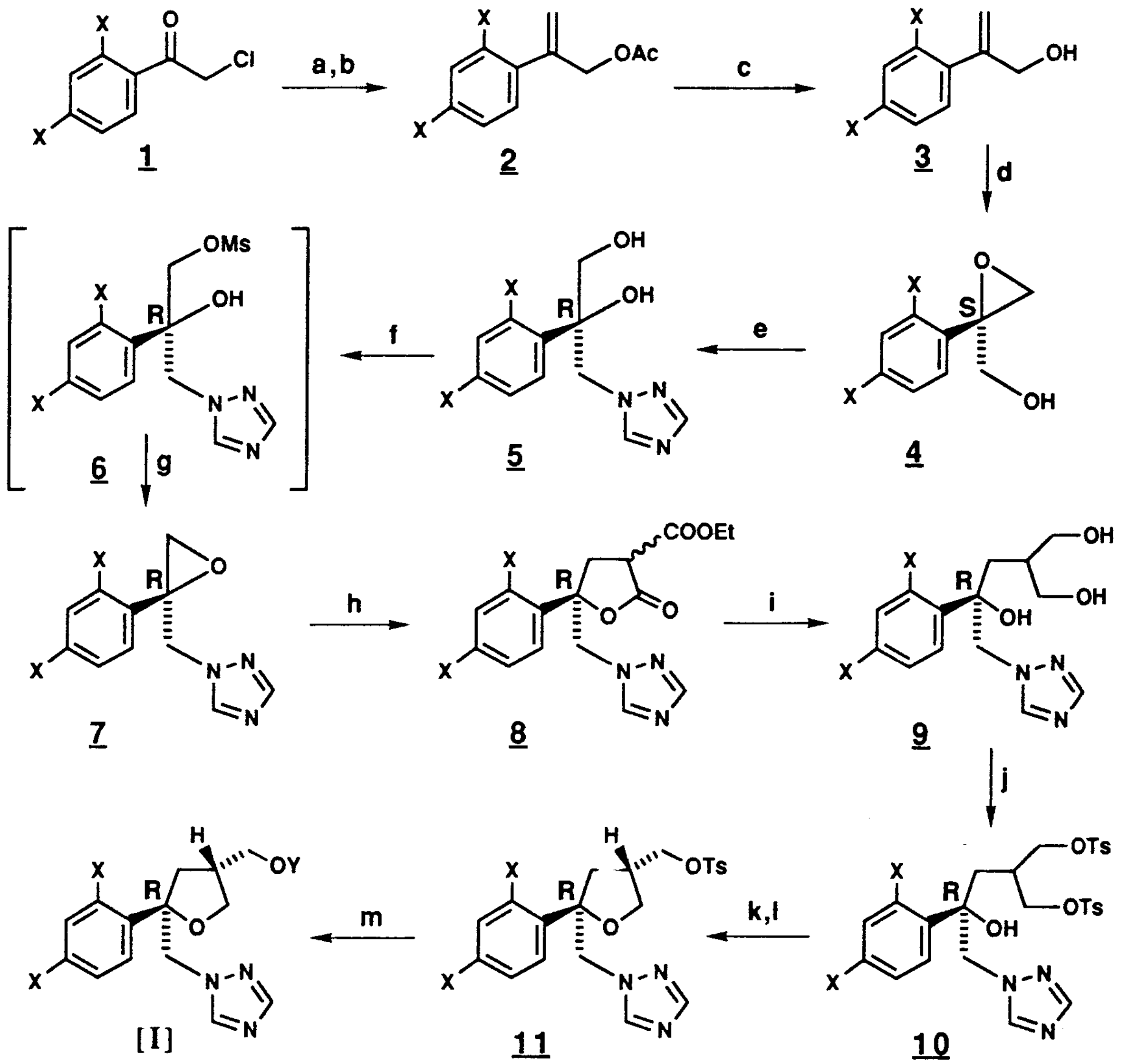


**Reagents:** (a) NaOAc; (b) Wittig Reaction; (c) KOH; (d) L-DET, TBHP, $(i-Pr)_4Ti$; (e) NaH, 1,2,4-triazole,DMF; (f) MsCl, $Et_3N$,$CH_2Cl_2$; (g) NaH, DMF; (h) NaH, $CH_2(COOEt)_2$, DMSO; (i) $LiBH_4$, EtOH; (j) TsCl, $Et_3N$, THF; (k) NaH, toluene, heat; (l) chromatography; (m) NaOY, DMSO

**X= F or Cl**

Scheme II

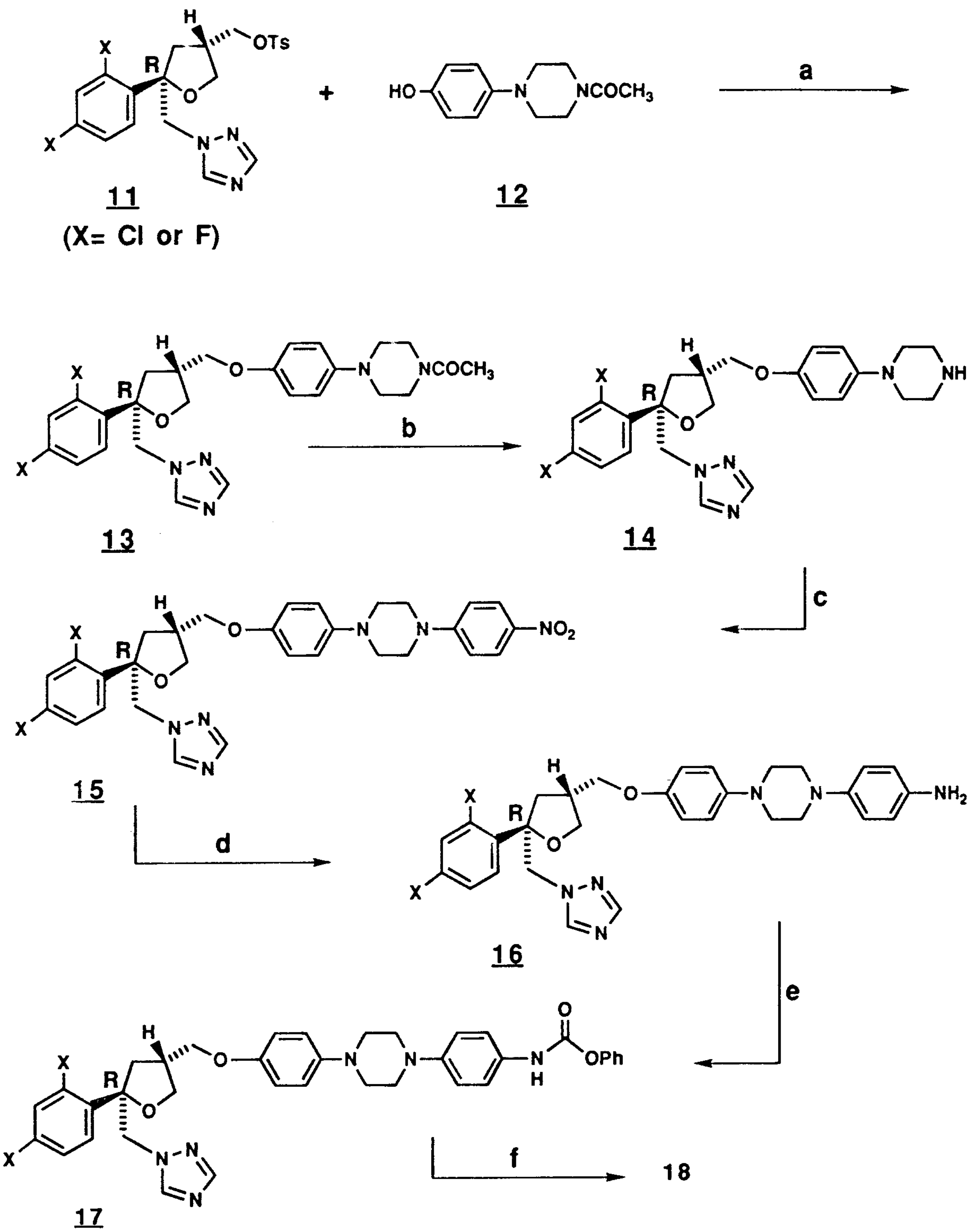


11 (X= Cl or F)

12

13

14

15

16

17

18

## Scheme II (cont'd.)

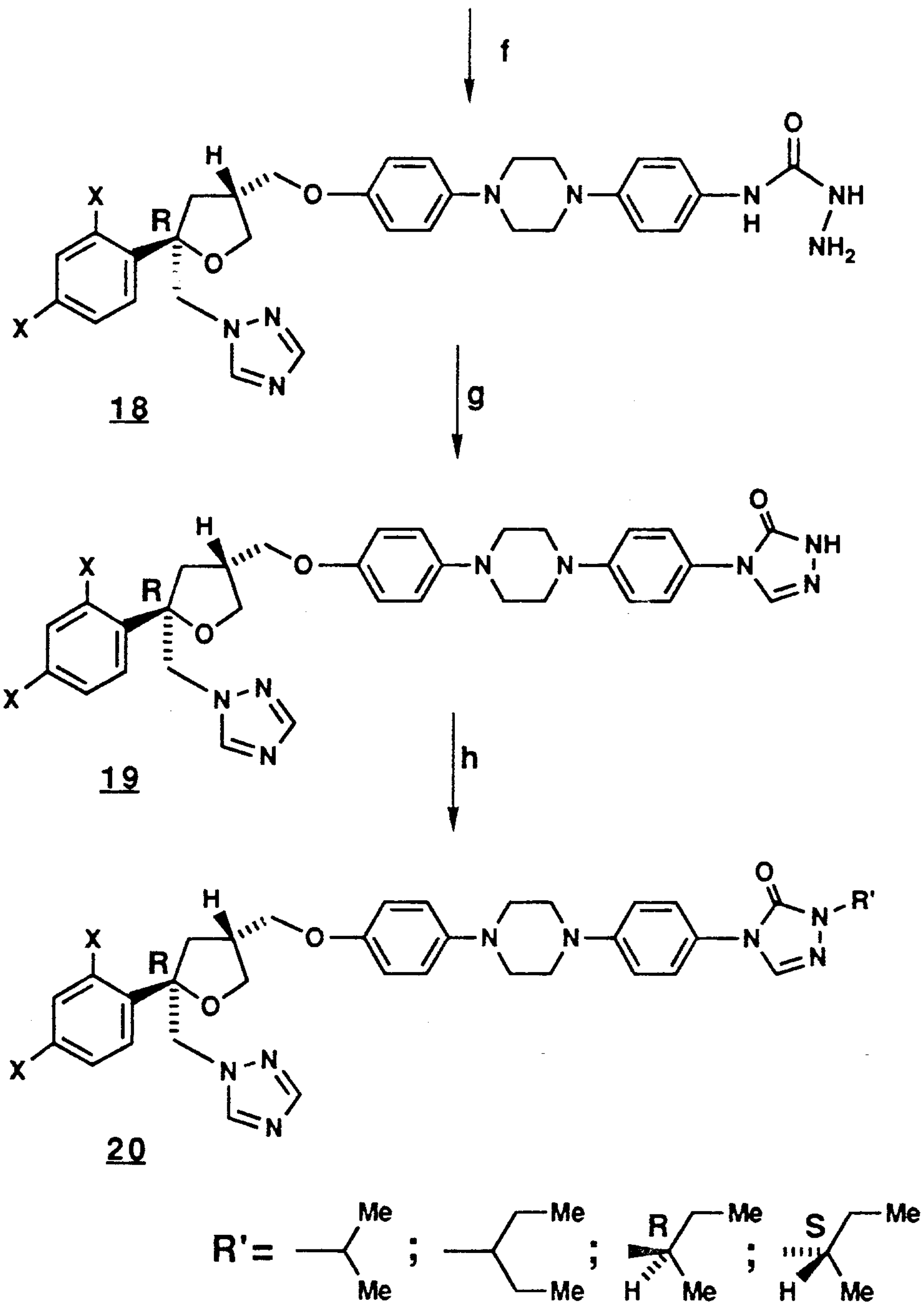


18

19

20

**Reagents:** (a) NaH; (b) NaOH/n – BuOH; (c) p – Cl – $C_6H_4NO_2$/ $K_2CO_3$/ DMSO; (d) $H_2$/Pt/C; (e) $C_6H_5OCOCl$/ pyridine/ $CH_2Cl_2$; (f) $NH_2.NH_2$/ $H_2O$/ dioxane; (g) Formamidine acetate/DMF/heat; (h) according to Examples 19 and 20

Scheme III

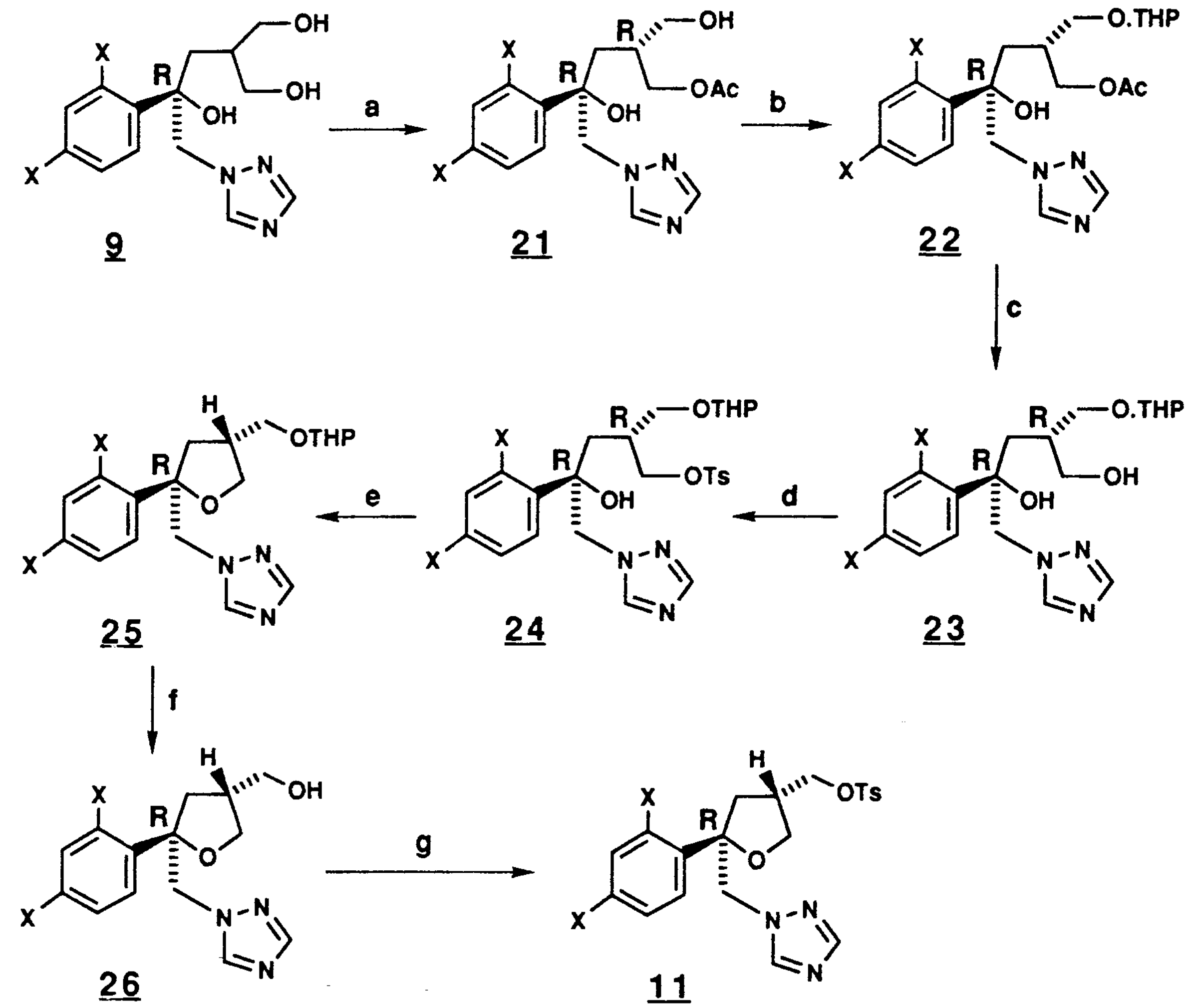


9 21 22 23 24 25 26 11

**Reagents:** (a) Porcine pancreatic lipase/EtoAc; (b) dihydropyran/$H^+$; (c) KOH; (d) Tosyl chloride/ pyridine; (e) NaH; (f) Methanol/$H^+$; (g) Tosyl chloride/ pyridine.

The compounds of formula I may be prepared by reaction of compound 11 (Compound of formula III wherein LG = OTs) with alcohols of formula HOY in the presence of a strong base, e.g., NaH in an aprotic solvent, such as DMSO.

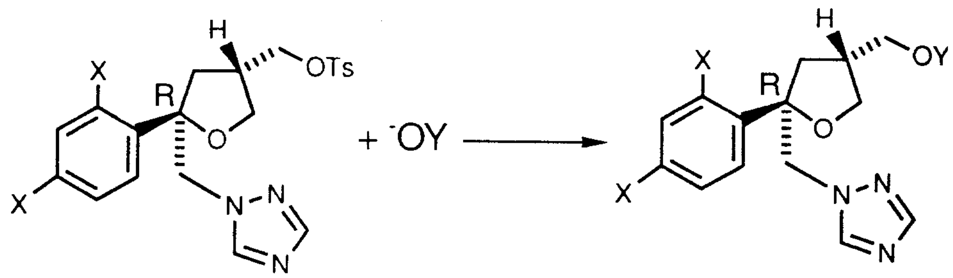

11 [I]

(R) – "Tosylate" Series
See Example 15
wherein

X = F or Cl;
Y =

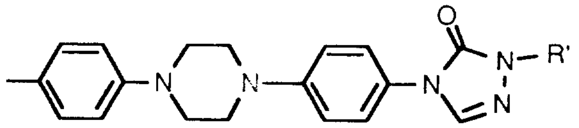
;

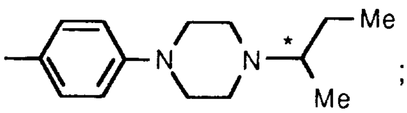
;
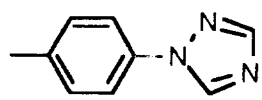
;

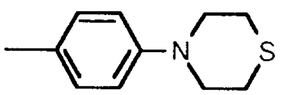
;
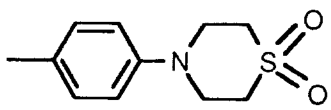
; or

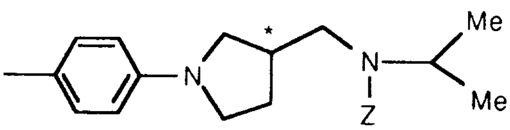

R' = ($C_1-C_{10}$) alkyl; ($C_2-C_{10}$) alkenyl; ($C_2-C_{10}$) alkynyl; ($C_3-C_8$) cycloalkyl; or $CH_2R^2$;
$R^2$ = ($C_1-C_3$) perhaloalkyl; $CO_2R^3$, $-{}^*CH(OR^4)CH_2OR^4$ or $CH_2N(R^5)_2$
$R^3$ = lower alkyl or H
$R^4$ = $R^3$ or $(CH_2)_2OR^3$
$R^5$ = lower alkyl

Z = H, or ($C_1-C_5$) alkanoyl and the carbons with the asterisks (*) have the R or S absolute configuration. Compound 11 is a preferred intermediate of the compounds represented by formula III wherein LG = OTs

The alcohols HOY are commercially available, or are prepared in accordance with published procedures or prepared in accordance with this invention. See Examples 27 and 28 for preparation of the intermediates of formula IV, V and VI.

The preferred compounds of this invention are represented by formula II

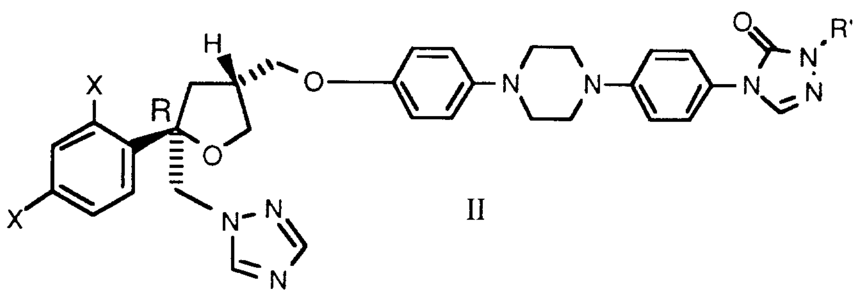

II

wherein X in both places is F or both Cl;
R' =

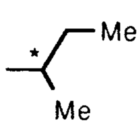
;
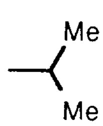
; or
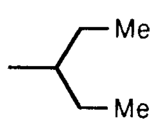
;

and the carbons with the asterisk (*) have the R or S absolute configuration.

The preferred antifungal compounds of this invention are represented by formulas IIa, IIb and IIc.

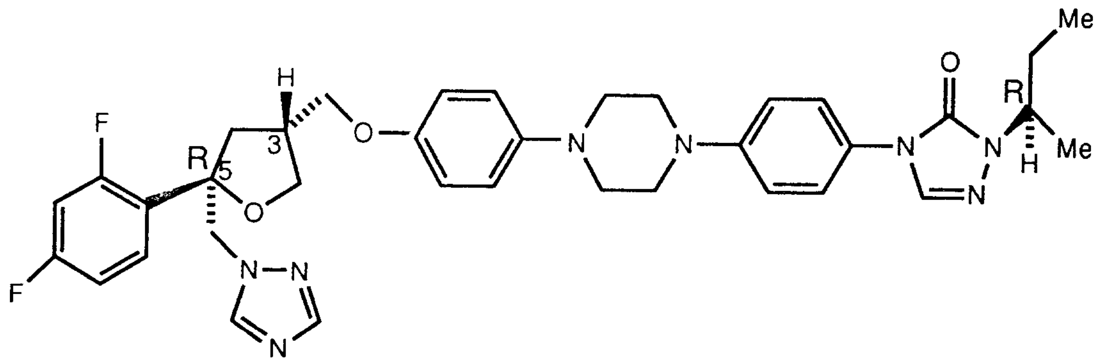

IIa

and which is named (−)−[(5R)−*cis*−[−4−[4−[4−[4−[[5−(2,4−difluorophenyl)−5−(1H−1,2,4−triazol−1−ylmethyl)tetrahydrofuran−3−yl]methoxy]phenyl]−1−piperazinyl]−2,4−dihydro−2[(R)−(1−methylpropyl)]−3H−1,2,4−triazol−3−one (See Example 23) and

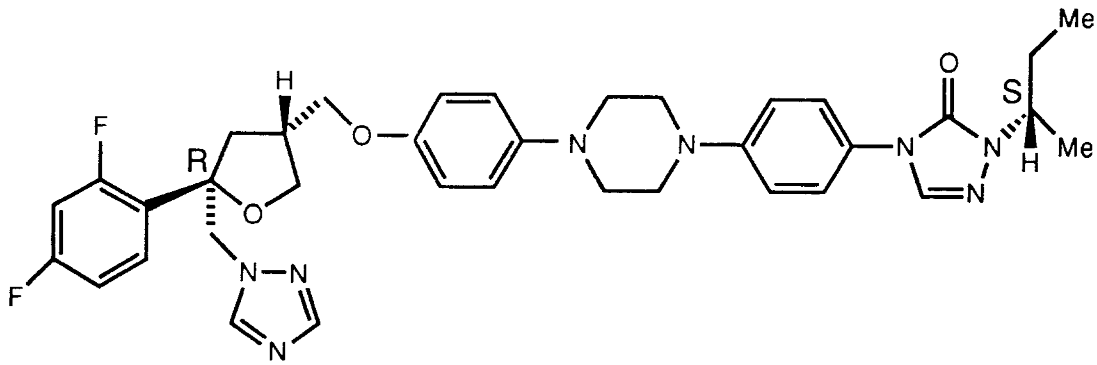

IIb

and which is named (−)−[(5R)−*cis*−[−4−[4−[4−[4−[[5−(2,4−difluorophenyl)−5−(1H−1,2,4−triazol−1−ylmethyl) tetrahydrofuran−3−yl]methoxy]phenyl]−1−piperazinyl]phenyl]−2,4−dihydro−2−[(S)−(1−methylpropyl)]−3H−1,2,4−triazol−3−one (See Example 24) and

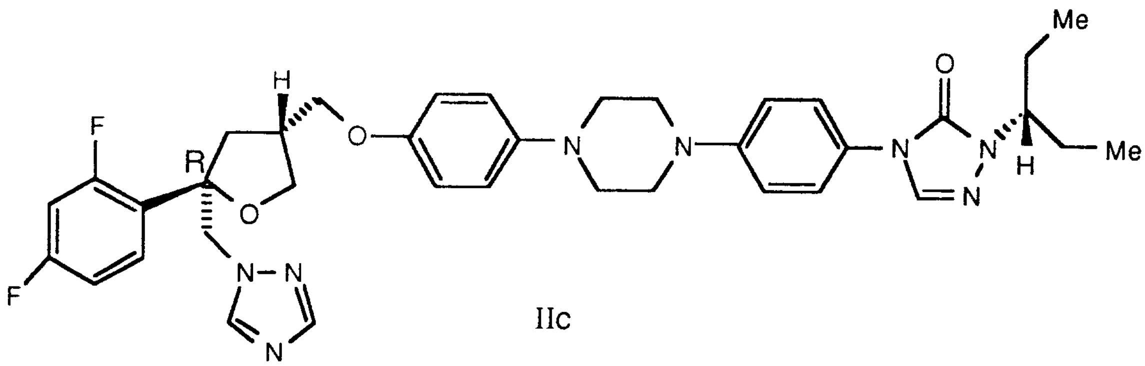

IIc

and which is named (−)−[(5R)−*cis*−[−4−[4−[4−[4−[[5−(2,4−difluorophenyl)−5−(1H−1,2,40−triazol−1−ylmethyl)tetrahydrofuran−3−yl]methoxy]phenyl]−1−piperazinyl]phenyl]−2,4−dihydro−2−(3−pentyl)]−3H−1,2,4−triazol−3−one (See Example 27.5)

The compound IIc is more preferred.

Compounds represented by formula I exhibit broad spectrum antifungal activity, in conventional antifungal screening tests, against human and animal pathogens, such as the following: *Aspergillus*, *Blastomyces, Candida*, *Cryptococcus*, *Coccidioides*, *Epidermophyton*, *Fonsecaea*, *Fusarium*, *Geotrichum*, *Histoplasma*, *Monosporium*, *Paracoccidioides*, *Rhodotorula*, *Saccharomyces*, *Torulopsis*,

*Trichophyton and others.*

The compounds of formula II are not inducers of various cytochrome P-450 liver drug metabolizing enzymes in an in vivo rat model.

The compounds of formula I exhibit topical, oral and parenteral antifungal activity in in vivo tests in animals and such activity is unexpectedly better than that of saperconazole and itraconazole as well as that of the compounds specifically disclosed by Saksena et al. in USP 5,039,676.

The antifungal compounds of formula I and pharmaceutical compositons of this invention are expected to exhibit anti-allergic, anti-inflammatory and immunomodulating activities, broad spectrum antiinfective activity, e.g., antibacterial, anti-protozoal and antihelminthic activities.

The present invention also provides a composition for treating or preventing fungal infections comprising an antifungally effective amount of a compound represented by formula I or a pharmaceutically acceptable salt thereof and a pharmaceutically acceptable carrier or diluent.

The pharmaceutical compositions of the present invention may also contain a fungicidally effective amount of other antifungal compounds such as cell wall active compound. The term "cell wall active compound", as used herein, means any compound that interferes with the fungal cell wall and includes, but is not limited to, compounds such as papulacandins, echinocandins, and aculeacins as well as fungal cell wall inhibitors such as nikkomycins, e.g, nikkomycin K and others which are described in USP 5,006,513 which is hereby incorporated by reference.

The preferred pharmaceutically acceptable acid addition salts are nontoxic acid addition salts formed by adding to the compounds of the present invention about a stoichiometric amount of a mineral acid, such as HCl, HBr, $H_2SO_4$, $HNO_3$ or $H_3PO_4$, or of an strongly ionized organic acid, such as trifluoro acetic, trichloroacetic, para-toluene sulfonic, methanesulfonic, and the like.

The pharmaceutical compositions of the present invention may be adapted for oral, parenteral, topical or vaginal administration They are formulated by combining the compound of formula I or an equivalent amount of a pharmaceutically acceptable salt of compound I with an suitable, inert, pharmaceutically acceptable carrier or diluent.

Examples of suitable compositions include solid or liquid compositions for oral administration such as tablets, capsules, pills, powders, granules, solutions, suppositories, suspensions or emulsions. A solid carrier can be one or more substances which may also act as diluents, flavoring agents, solubilizers, lubricants, suspending agents, binders or tablet disintegrating agents; it can also be an encapsulating material. In powders, the carrier is a finely divided solid which is in admixture with the finely divided active compound. In the tablet, the active compound is mixed with carrier having the necessary binding properties in suitable proportions and compacted in the shape and size desired.

Topical dosage forms may be prepared according to procedures well known in the art, and may contain a variety of ingredients, excipients and additives. The formulations for topical use include ointments, creams, lotions, powders, aerosols, pessaries and sprays.

For preparing suppositories, a low melting wax such as a mixture of fatty acid glycerides or cocoa butter is first melted, and the active ingredients are dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection. Liquid preparations can also be formulated in solution with an appropriate amount of a hydroxypropyl $\alpha$- $\beta$- or -$\gamma$-cyclodextrin having 2 to 11 hydroxypropyl groups per molecule of cyclodextrin, polyethylene glycol, e.g., PEG-200 or propylene glycol, which solutions may also contain water. Aqueous solutions suitable for oral use can be prepared by adding the active component in water and adding suitable colorants, flavors, stabilizing, sweetening, solubilizing and thickening agents as desired. Aqueous suspensions suitable for oral use can be made by dispersing the active component in finely divided form in water. A particularly preferred aqueous pharmaceutical composition may be prepared from the compounds of formula I or IIa or IIb together with hydroxypropyl-$\beta$-cyclodextrin in water. The use of derivatives of $\alpha$-, $\beta$- and $\gamma$-cyclodextrins, for example, hydroxpropyl-$\beta$-cyclodextrin are disclosed by N. Bodor USP 4,983,586, Pitha USP 4,727,064 and Janssen Pharmaceutical International Patent Application No. PCT/EP 84/00417.

The pharmaceutical compositions of the present invention may be prepared by admixing the pharmaceutically acceptable carrier e.g. a hydroxypropyl-$\beta$-cyclodextrin in water, and adding thereto an antifungally effective amount of a drug of the present invention. The solution so formed is filtered, and optionally, the water may be removed by well known methods, e.g., rotatory evaporation or lyophilization. The formation of the solution may take place at a temperature of about 15° to 35°C. The water is normally sterilized water and may also contain pharmaceutically acceptable salts and buffers, e.g., phosphate or

citrate as well as preservatives. The molar ratio of the antifungal compound of formula I to hydroxpropyl-β-cyclodextrin is about 1:1 to 1:80, preferably 1:1 to 1:2. Normally the hydroxypropyl-β-cyclodextrin is present in molar excess.

Also included are solid form preparations which are intended to be converted, shortly before use, into liquid form preparations for either oral or parenteral administration. The solid form preparations intended to be converted to liquid form may contain, in addition, to the active materials, such as compounds of this invention, and optionally a cell wall active compound, especially a fungal cell wall inhibitor, e.g., a nikkomycin, flavorants, colorants, stabilizers, buffers, artificial and natural sweeteners, dispersants, thickeners, solubilizing agents and the like. The solvent utilized for preparing the liquid form preparations may be water, isotonic water, ethanol, glycerin, polyethylene glycols, propylene glycol, and the like, as well as mixtures thereof.

Parenteral forms to be injected intravenously, intramuscularly, or subcutaneously are usually in the form of a sterile solution, and may contain salts or glucose to make the solution isotonic.

The topical dosage for humans for antifungal use in the form of a pharmaceutical formulation comprising a compound of formula I (usually in the concentration in the range from about 0.1% to about 20% preferably from about 0.5% to about 10% by weight) together with a non-toxic, pharmaceutically acceptable topical carrier, is applied daily to the affected skin until the condition has improved.

In general, the oral dosage for humans for antifungal use ranges from about 1 mg per kilogram of body weight to about 50 mg per kilogram of body weight per day, in single or divided doses, with about 2 mg per kilogram of body weight to about 20 mg per kilogram of body weight per day being preferred.

In general, the parenteral dosage for humans for antifungal use ranges from about 0.5 mg per kilogram of body weight per day, to about 20 mg kilogram of body weight per day, in single or divided doses, with about 1 to about 10 mg per kilogram of body weight per day being preferred.

GENERAL EXPERIMENTAL

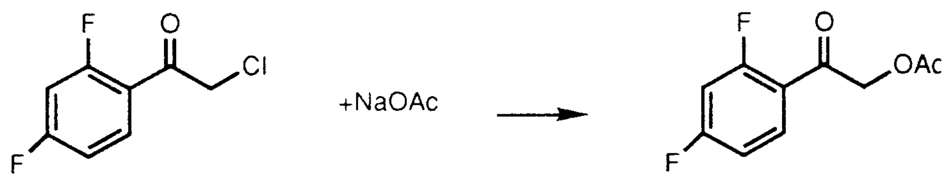


EXAMPLE 1a

2-Acetyloxy-1-(2,4-difluorophenyl)ethanone

Add 191 g of 2-chloro-2',4'-difluoroacetophenone (Aldrich Chemical Co.) to a mixture of 246 g of sodium acetate, 3 g of NaI, and 3 L of DMF. Stir the mixture at 20 °C for 18 hr. then concentrate it to 1 L. Pour the residue into 6 L of cold dilute aqueous HCl and extract with EtOAc. Wash the extract with brine, dry it over anhydrous $Na_2SO_4$, filter the so-formed mixture, and evaporate the filtrate to leave a residue. Chromatograph the residue on silica gel, eluting with $CH_2Cl_2$-hexane to obtain 198 g of the title compound.

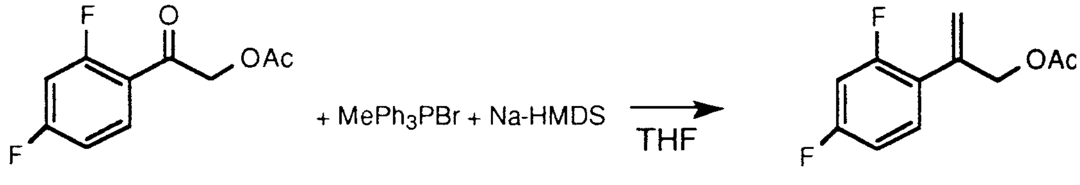

EXAMPLE 1b

1 – [2 – (2,4 – Difluorophenyl)] – 2 – propenol acetate

Suspend 131 g of $MePh_3PBr$ in 270 mL of mechanically – stirred, dry THF at 20 ° C. Add 393 mL of 1M $NaN(Me_3Si)_2$ in THF, slowly at first, then rapidly over 5 min. while applying just enough ice cooling to maintain the temperature at < 23 ° C. Stir the so – formed mixture for 1 hr at 20 ° – 24 ° C, cool it to ~ – 70 ° C, and stir it another 1/2 hr. Then add thereto a solution of 65.5 g of the product of Example 1a in 140 mL of dry THF, at a rate slow enough to keep the temperature below – 70 ° C. Continue to stir the so – formed reaction mixture in the cold bath overnight during which the temperature rises to 20 ° C. Add 50 mL of EtOAc to the so – formed suspension, and then add 3 L of hexane. Allow the so – formed mixture to stand for ~15 min., and suction – filter to remove $Ph_3PO$. While the filter cake is still damp, transfer it to a beaker. Triturate the cake thoroughly with 1/2 L of hexane and suction – filter again to remove the remainder of product. Wash the combined hexane filtrates with 2 x 1 L of a 1:1 (v/v) MeOH – water, and then with brine. Dry the organic layer over $MgSO_4$, filter and evaporate the filtrate to leave a red oil. Add 1.5 L of hexane and suction – filter through a Celite pad to leave a clear yellow solution. Chromatograph the yellow oil on silica gel, eluting with 1/2 L of hexane, then 1L of 15:1 (v/v) hexane – EtOAc. Combine the homogeneous fractions to yield 38.6 g of the title compound as an oil.

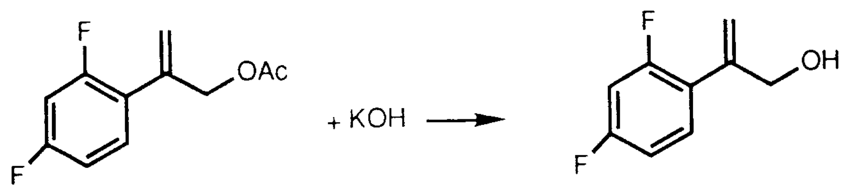


EXAMPLE 1c

2 – (2,4 – Difluorophenyl) – 2 – propenol.

Dissolve 40 g of the product of Example 1b in 400 mL of dioxane. Add a solution of 18 g of 85% KOH in 315 mL of water. Stir the so – formed mixture vigorously for 1 hr, and then pour the mixture into 1 L of $Et_2O$. Separate the aqueous layer and extract it with 250 mL of $Et_2O$. Combine the organic extracts, and wash them with water and then brine. Dry the organic extract over anhydrous $K_2CO_3$, and add 10 g of charcoal thereto. Filter, and evaporate the filtrate to leave 31.3 g of the title compound as a straw – colored oil.

EXAMPLE 1d

(S) – ( – ) – [2 – [2 – (2,4 – Difluorophenyl)]oxiranyl]methanol

Add 33g of activated 3Å molecular sieve powder to a solution of 13g of L – ( + ) – diethyl tartarate in 2.3L of $CH_2Cl_2$, and cool the so – formed mixture to – 5 ° C. Add a solution of 15.4 mL of titanium tetra – isopropoxide in 100 mL of $CH_2Cl_2$ over 2 – 3 minutes and then cool the so – formed mixture to – 22 ° C. Add 109.5 mL of a 5.5 M solution of tert – butylhydroperoxide in 2,2,4 – trimethylpentane over 4 – 6 minutes, and cool the so – formed mixture to – 25 ° C. Stir the mixture at – 25 ° C for 25 minutes and then add a solution of 40g of 2 – (2,4 – difluorophenyl) – 3 – propenol of Example 1c in 100 mL of $CH_2Cl_2$ over 3 – 4 minutes. Stir the so – formed mixture at – 27 ° C for 4 1/2 hour. Add 102 mL of 30% aqueous sodium hydroxide saturated with NaCl and stir the so – formed mixture while warming to + 10 ° C over a 1/2 hour period. Add thereto 100 g of anhydrous $MgSO_4$ and 33g of Celite, and stir 1/2 hour at + 10 ° C. Suction – filter the mixture, wash the so – formed filter cake with 1.2 L of diethyl ether ($Et_2O$) and then 1.5L of toluene, and dry the combined organic layers over anhydrous $MgSO_4$. Filter the organic layer, and evaporate the filtrate in vacuo to form a residue. Dissolve the residue in 1L of $Et_2O$ and suction – filter the mixture to remove insolubles. Suction – filter the filtrate through 100g of silica gel, and wash the pad with 200 mL of fresh $Et_2O$. Evaporate the filtrate in vacuo to give 41g (94%) of the crude title compound as a yellowish oil, $[\alpha]_D^{25}$ – 36.7 ° (c = l, MeOH); PMR ($CDCl_3$) δ 7.40(m, 1H), 6.85(m, 2H), 3.95(m,2H), 3.31(d,1H), 2.84 (d,1H), 1.91(m,1H, deuterium exchangeable).

EXAMPLE 2

(R) – ( + ) – [2 – [2 – (2,4 – Difluorophenyl)]oxiranyl]methanol

Follow the procedure of Example 1d, except substitute an equivalent amount of D – ( – ) diethyl tartarate in place of L – ( + ) diethyl tartarate to give the crude title compound, $[\alpha]_D^{25}$ + 33.9° (c = l, MeOH).

Purify a portion of the crude compound by silica gel chromatography to obtain a sample homogeneous by TLC, $[\alpha]_D^{25}$ + 40.0° (c = l, MeOH)

EXAMPLE 3

(R) – ( – ) – 2 – (2,4 – Difluorophenyl) – 3 – (1,2,4 – triazol – 1 – yl) – 1,2 – propanediol

Dissolve 8.91g of 1H – 1,2,4 – triazole in 150 mL of anhydrous DMF and cool to 0 – 5° C. Add 2.81g of sodium hydride (60% oil dispersion) and stir the so – formed mixture 30 minutes at room temperature. Add thereto 10.9 g of the product of Example 1d. Stir the so – formed reaction mixture for 2 hours at 60 – 70° C. Cool the mixture to room temperature, add thereto 10 ml of $H_2O$ and evaporate it in vacuo to give a residue. Dissolve the residue in 100 mL of $H_2O$ and 900 ml of ethyl acetate (EtOAc). Extract the $H_2O$ layer with another 250 mL of EtOAc. Wash the combined EtOAc extracts with 100 mL of brine. Dry the EtOAc extracts over anhydrous $MgSO_4$ and evaporate. Triturate the so – formed oily residue with 10 mL of $CH_2Cl_2$ and add 100 mL of $Et_2O$. Stir the $CH_2Cl_2$ – $Et_2O$ mixture for 1 hour at room temperature. Filter to give 11.2g (75%) of the title compound, $[\alpha]_D^{25}$ – 70.7 (C = 10, MeOH), mass spectrum (FAB): 256 (M + H $^{\oplus}$).Recrystallize 1.0g of the filtered product from 5 mL of $CH_3CN$ to give 0.83g of the title compound, m.p. 99 – 100° C; $[\alpha]_D^{25}$ – 71.5° (C = 1.0, MeOH); elemental analysis: Calculated for $C_{11}H_{11}F_2N_3O_2 1/2CH_3CN$; 52.27C, 4.57H, 17.78N, 13.78F; Found: 52.26C, 4.58H, 17.54N, 13.78F; PMR(DMSO) δ 8.25 (s,1), 7.66(s,1), 7.33, (m,1), 7.09(t,1), 6.90(t,1), 5.72(s,1), 5.05(t,1), 4.53(s,2), 3.61(m,2).

EXAMPLE 4

(S) – ( + ) – 2 – (2,4 – Difluorophenyl) – 3 – (1,2,4 – triazol – 1 – yl) – 1,2 – propanediol

Follow the procedure of Example 3, except substitute an equivalent quantity of the product of Example 2 in place of the product of example 1 to give the title compound; MP. 95 – 101° C; $[\alpha]_D^{25}$ + 70.0° (c = 1.0, MeOH). The PMR and Mass spectra were consistent with the structure of the title compound.

EXAMPLE 5

(R) – 2 – (2,4 – Difluorophenyl) – 3 – (1,2,4 – triazol – 1 – yl) – 1,2propanediol – 1 – methanesulfonate

Suspend 10.9 g of the powdered product of Example 3 in 150 mL of $CH_2Cl_2$. Add thereto 8.95 mL of triethylamine and cool to the so – formed mixture 0 – 5° C. Add 3.64 mL of methanesulfonyl chloride in 20 ml of $CH_2Cl_2$ over 10 min. Stir the so – formed mixture for 1 hour at room temperature. Cool it to 0 – 5° C, extract with 100 mL of cold (0 – 5° C) 5% $KH_2PO_4$, followed by 100 mL of cold (0 – 5° C) $H_2O$, followed by 50 mL of brine. Dry the separated organic layer over anhydrous $MgSO_4$ and evaporate to obtain 13.7 g (96%) of the title compound. Mass spectrum (FAB): 333 ($M + H^+$); PMR ($CDCl_3$) δ 7.95 (s,1), 7.82 (s,1), 7.53(m,1), 6.81(m,2), 4.84(d,1), 4.65(d,1), 4.46(m,2), 3.05(s,3).

EXAMPLE 6

(S) – 2 – (2,4 – Difluorophenyl) – 3 – (1,2,4 – triazol – 1 – yl) – 1,2 – propanediol – 1 – methanesulfonate

Follow the procedure of Example 5, except substitute an equivalent quantity of the product of Example 4 in place of the product of example 3 to give the title compound . The PMR is consistent with the structure of the title compound.

EXAMPLE 7

(R)-1-[2-[2-[2,4-Difluorophenyl)]oxiranylmethyl]-1,2,4-triazole

Dissolve 13.7g of the product of Example 5 in 200 mL of anhydrous DMF and cool the so-formed solution to 10-15 °C. Add thereto 1.71g of sodium hydride (60% oil dispersion) and stir the so-formed reaction mixture at room temperature for 90 minutes. Concentrate in vacuo to 50 mL. Add thereto 200 mL of cold $H_2O$ (0-5 °C) and extract with 3 x200 mL portions of EtOAc. Wash the combined EtOAc extracts with 100 mL of brine. Dry the EtOAc extracts over anhydrous $MgSO_4$ and evaporate it to give 10.8g of a residue. Apply the residue in $CH_2Cl_2$ to a column of 400 g of MPLC grade silicon gel previously prepared by slurry packing with $CH_2Cl_2$ containing 1 mL of $Et_3N$ per liter. Elute with 1 liter, each of 25, 50 and 75% EtOAc; $CH_2Cl_2$ (v/v) followed by 2 liters of EtOAc. Combine the fractions to give 6.92g (68%) of the title compound. Mass spectrum (FAB): 238 ($M+H^+$); PMR ($CDCl_3$) $\delta$ 7.97(s,1), 7.77(s,1), 7.07(m,1), 6.73(m,2); 4.73(d,1), 4.41(d,1), 2.84(d,1), 2.78(d,1).

EXAMPLE 8

(S)-1-[2-[2-(2,4-difluorophenyl]oxiranylmethyl]-1,2,4-triazole

Follow the procedure of Example 7, except substitute an equivalent amount of the product of Example 6 in place of the product of Example 5 to give the title compound. [PMR is consistent with the structure of the title compound].

EXAMPLE 9

Ethyl(5R-cis)-, and (5R-trans)-5-(2,4-Difluorophenyl)-2-oxo-5-[(1H-1,2,4-triazol-1-yl)-methyl]tetrahydro-3-furancarboxylate

Dissolve 9.35 mL of diethyl malonate in 70 mL of anhydrous DMSO. Add 2.24g of sodium hydride (60% oil dispersion) in 2 portions and stir the so-formed reaction mixture at room temperature for 1 hour. Add 6.65 g of the product of Example 7 and stir 18 hours at 50-55 °C. Cool to room temperature and pour the reaction mixture into a well-stirred mixture of 500 mL of $KH_2PO_4$, 500 mL of brine, and 1 liter of EtOAc. Separate and extract the $H_2O$ layer with another 300 mL of EtOAc. Wash the combined EtOAc extracts with 500 mL of brine, Dry the EtOAc extracts over anhydrous $MgSO_4$ and evaporate to give an oil. Apply the oil with $CH_2Cl_2$ to a column of 400 g MPLC grade silica gel prepared with hexane. Elute with 500 mL of hexane, followed by 2 liters of 50% EtOAc: hexane (v/v), followed by 2 liters of EtOAc. Combine fractions to give 8.66g (80%) of the title compound. Mass spectrum (FAB): 352($M+H^{\oplus}$), PMR ($CDCl_3$) $\delta$ 8.08(s,2), 7.91(s,1), 7.71(s,1), 7.42(m,1), 7.13(m,1), 7.85(m,2), 4.60(m,4), 4.10(m,4), 3.49(t,1), 3.14(t,1), 3.89(m,4), 1.18(m,6).

EXAMPLE 10

Ethyl(5S-cis), and (5S-trans)-5-(2,4-Difluorophenyl)-2-oxo-5-(1H-1,2,4-triazol-1-yl)methyl]-tetrahydro-3-furancarboxylate

Follow the procedure of Example 9, except substitute an equivalent amount of the product of Example 8 in place of the product of Example 7 to give the title compound. [PMR is consistent with the structure of the title compound].

EXAMPLE 11

(R)-(-)-4-(2,4-Difluorophenyl)-2-hydroxymethyl-5-[1H-(1,2,4-triazol-1-yl)]-1,4-pentanediol

Dissolve 8.5 g of the product of Example 9 in 125 mL of EtOH and add 2.15 g of LiCl. Cool the stirred mixture to 0 °C and add 1.92 g of $NaBH_4$ in portions. Stir the mixture for 18 hr without further cooling. Add 125 mL of MeOH and 10 mL of $H_2O$ to the mixture and stir for 4 hr. Evaporate the mixture to dryness and extract the precipitate with warm EtOH. Evaporate the extract to dryness, add 200 mL of THF to the residue, and sonicate the stirred mixture for 15 min. Filter the mixture and evaporate the filtrate.

Chromatograph the residue on silica gel, eluting with $CH_2Cl_2$ – MeOH – $NH_4OH$ (95:5:1) v/v/v) to obtain 3.9 g of the title compound. Mass spectrum (FAB): 314 ($M + H^+$); PMR (DMSO) δ 8.25(s,1), 7.69(s,1), 7.35(m,1), 7.13(m,1), 6.94(m,1), 6.27(s,1), 5.16(t,1), 4.44(m,4), 3.39(m,1), 3.20(m,1), 3.05(t,2), 2.11(m,1), 1.52(m,1).

EXAMPLE 12

(S) – ( + ) – 4 – (2,4 – Difluorophenyl) – 2 – hydroxymethyl – 5 – [1H – (1,2,4 – triazolyl)] – 1,4 – pentanediol

Follow the procedure of Example 11, except substitute an equivalent amount of the product of Example 10 in place of the product of Example 9 to give the title compound. Chromatograph a portion of the crude product on silica gel eluting with $CH_2Cl_2$ – MeOH – $NH_4OH$ to give a product homogeneous by TLC. Dissolve the material in $H_2O$ and filter, and lyophilize the filtrate to give the title compound. $[\alpha]_D^{25}$ + 54.5 (c = 1.0, MeOH)

EXAMPLE 13

(R) – ( – ) – 4 – (2,4 – Difluorophenyl) – 2 – [[(4 – methylphenyl) – sulfonyloxy]methyl] – 5 – [1H – (1,2,4 – triazolyl)] – 1,4 – pentanediol – 1 – (4 – methylbenzene)sulfonate

Dissolve 4.4g of the product of Example 11 in 50 mL of $CH_2Cl_2$ – THF (1:1, v/v). Add 4.7 mL of $Et_3N$ and 180 mg of N,N – dimethylaminopyridine, and cool the solution to 0 ° C. Add thereto 5.9 g of p – toluenesulfonyl chloride in portions and stir the so – formed reaction mixture at 0 ° C for 1/2 hour, and then stir it at room temperature for 5 hours. Add 100 mL of EtOAc and suction – filter the mixture. Concentrate the filtrate; add thereto 150 mL of EtOAc, and wash with 5% aqueous $KH_2PO_4$. Wash the organic layer with cold aqueous 5% $NaHCO_3$, then with saturated brine, and then dry it over anhydrous $MgSO_4$. Filter the mixture, and evaporate the filtrate. Chromatograph the residue on silica gel, eluting with EtOAC – hexane to give 6.4 g (73%) of the title compound, PMR ($CDCl_3$ ) δ 7.95(s,1), 7.67(m,5), 7.30(m,6) 6.70(t,2), 4.74(d,1), 4.53(d,1), 4.13(m,1), 3.97(m,1), 3.8(m,2), 2.43(s,6), 1.95(m,2), 1.77(m,1). Mass spectrum (FAB): 622 ($M + H^+$).

EXAMPLE 14

(S) – ( + ) – 4 – (2,4 – Difluorophenyl) – 2 – [[(4 – methylphenyl) – sulfonyloxy]methyl] – 5 – [1H – (1,2,4 – triazolyl] – 1,4 – pentanediol – 1(4 – methylbenzene)sulfonate

Follow the procedure of Example 13 except substitute an equivalent amount of the product of Example 12 in place of the product of Example 11 to obtain the title compound, $[\alpha]_D^{25}$ + 14.2 ° (c = 1, MeOH).

EXAMPLE 15

( – ) – (5R – cis) – 5 – (2,4 – Difluorophenyl) – 5 – [(1H – 1,2,4 – triazol – 1 – yl)methyl] – tetrahydro – 3 – furanmethanol,4 – toluenesulphonate

Dissolve 6.3g of the product of Example 13 in 150 mL of toluene and heat the so – formed solution to 100 ° C. Add 2.4g of 60% NaH dispersion in oil portionwise, and then heat the so – formed reaction mixture at reflux until cyclization is complete (approx. 3 – 4 hours). Cool the mixture and decant the solution from excess NaH. Wash the solution with cold 5% aqueous $KH_2PO_4$. Evaporate the organic layer to form a residue and chromatograph the residue on silica gel, eluting with acetone – hexane to obtain 1.6g (35%) of the title compound as the less polar of the two products, $[\alpha]_D^{25}$ – 39.4 ° (c = 1, $CHCl_3$); PMR ($CDCl_3$) δ 8.09 (s,1), 7.88 (m,3), 7.31 (m,3), 6.81(m,2), 4.52(ABq,2), 3.99(m,1), 3.85(m,1), 3.70(m,1), 3.59(m,1), 2.49(m,2), 2.47(s,3), 1.90(m,1). Mass spectrum (FAB): 450 ($M + H^+$).

EXAMPLE 16

(+)-(5S-cis)-5-(2,4-Difluorophenyl)-5-[(1H-1,2,4-triazol-1-yl)methyl]-tetrahydro-3-furanmethanol,4-toluenesulphonate

Follow the procedure of Example 15, except substitute an equivalent amount of the product of Example 14 in place of the product of Example 13 to give the title compound, $[\alpha]_D^{25}$ + 40.3° (c=0.3, $CHCl_3$), mp 96-98°C.

EXAMPLE 17

S-(+)-2-Butanol tosylate

Dissolve 57.07 g of S-(+)-2-butanol in 600 mL of dry pyridine. Cool to 0°-5°C. Add thereto 161.44 g of p-toluenesulfonyl chloride, portionwise, at 0°-5°C with stirring and under dry $N_2$. Stir the so-formed mixture at 0°-5°C for two days. Evaporate the pyridine at 30°-35°C under high vacuum. Dissolve the so-formed residue in 1 L of $Et_2O$ ether and 750 mL of $H_2O$. Wash the organic layer with 1NHCl, then with 5% $Na_2CO_3$, and then with saturated brine. Dry the organic layer over anhydrous $MgSO_4$. Filter the mixture and evaporate the filtrate to give 174g (99%) of the title compound, $[\alpha]_D^{25}$ = + 10.53° (c=1, MeOH).

EXAMPLE 18

R-(-)-2-Butanol tosylate

Follow the procedure of Example 17, except substitute an equivalent quantity of R-(-)-2-butanol in place of S-(+)-2-butanol to obtain the title compound. $[\alpha]_D^{25}$ - 11.97° (C=1, MeOH).

EXAMPLE 19

R(-)-2,4-Dihydro-4-[4-[4-(4-methoxyphenyl)-1-piperazinyl]-phenyl]-2-(1-methylpropyl)-3H-1,2,4-triazole-3-one

Dissolve 18.9 g of the product of Example 17 in 450 mL of dry DMSO. Add 22.5 g of 2,4-dihyro-4-[4-[4-(4-methoxyphenyl)-1-piperazinyl]phenyl]-3H-1,2,4-triazole-3-one prepared as described by J. Heeres et al J. Med Chem (1984) 27, 894-900 followed by 5.3 g of powdered KOH. Stir the so-formed suspension at room temperature and under dry $N_2$ for 4 days. Pour the suspension into 4.5 liter of ice-water. Filter the so-formed precipitate and wash it with $H_2O$. Chromatograph the residue on silica gel, eluting with $CH_2Cl_2$-acetone to give 9.79 g (36%) of the title compound, $[\alpha]_D^{25}$ - 5.56° (c=1, $CHCl_3$).

EXAMPLE 20

(S(+)-2,4-Dihydro-4-[4-[4-[4-methoxyphenyl)-1-piperazinyl]-phenyl]-2-(1-methylpropyl)-3H-1,2,4-triazole-3-one and the 2-(1-methylethyl) and 2-(1-methylbutyl)-substituted 3H-1,2,4-triazole-3-one analogs thereof

Follow the procedure of Example 19 except substitute an equivalent quantity of the compound of column A (Example 18) below in place of S-(+)-2-butanol tosylate to obtain the product in column B below.

| Column A | Column B |
| --- | --- |
| 1. 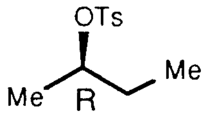 | 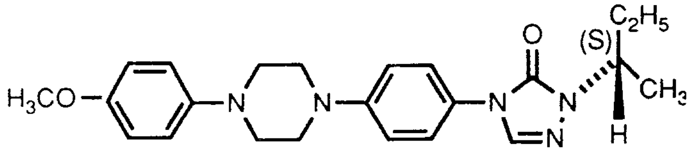 |
| 2. 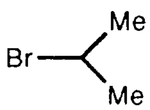 | 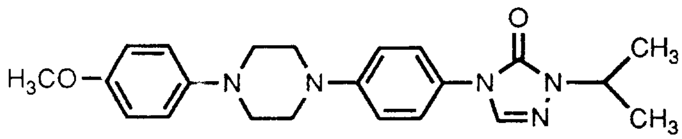 |
| 3. 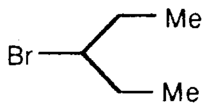 | 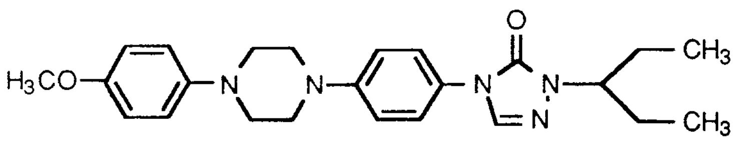 |

EXAMPLE 21

R-(-)-2,4-Dihydro-4-[4-[4-(4-hydroxyphenyl)]-1-piperazinyl]-phenyl]-2-(1-methylpropyl)-3H-1,2,4-triazole-3-one.

Suspend 9.7 of the product of Example 19 in 150 mL of aqueous 48% HBr solution. Reflux the so-formed mixture overnight. Cool the reaction mixture until a precipitate is formed. Add the so-formed slurry slowly to a saturated aqueous $NaHCO_3$ solution. Filter the precipitate and wash with EtOAc-hexane. Recrystalize the filtered solid from $CH_3CN$ to give 7.3g (78%) of the title compound, $[\alpha]_D^{25}$ - 5.29° (c=1, $CHCl_3$).

EXAMPLE 22

Follow the procedure of Example 21 except substitute an equivalent quantity of the appropriate product of Example 20 in place of the product of Example 19 to obtain the corresponding demethylated products as shown below:

1. 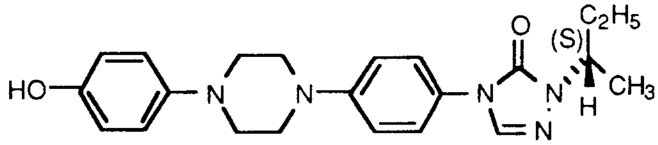

2. 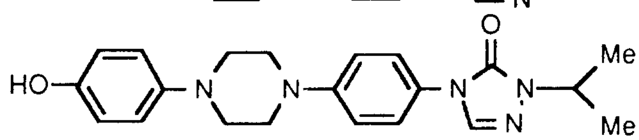

3. 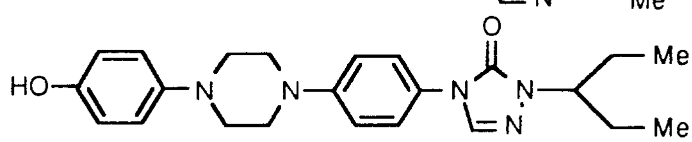

EXAMPLE 23

(−)−[(5R)−cis]−4−[4−[4−[4−[[5−(2,4−Difluorophenyl)−5−(1H−1,2,4−triazol−1−ylmethyl)−tetrahydrofuran−3−yl]methoxy]phenyl[−1−piperazinyl]phenyl]−2,4−dihydro−2−[(1R)−(1−methylpropyl)]−3H−1,2,4−triazol−3−one.

Dissolve 2.9g of the product of Example 21 in 70 mL of dry DMSO. Add thereto 0.32g of a 60% NaH dispersion in oil, heat the so−formed reaction mixture to 60°C, and stir for 30 minutes. Add 3.3g of the product of Example 15; heat the so−formed reaction mixture to 80°C, and stir for 45 minutes. Pour the hot mixture into 700 mL of ice−water containing 1/2g of $K_2CO_3$. Stir for 10 minutes, then suction−filter, and dry the so−formed precipitate. Dissolve the precipitate in $CH_2Cl_2$ and chromatograph the so−formed solution on silica gel, eluting with acetone−$CH_2Cl_2$ to give 4.18g (85%), $[\alpha]_D^{25}$ − 28.3° (c=1, $CHCl_3$); PMR ($CDCl_3$) δ 8.13(s,1), 7.81(s,1), 7.63(s,1), 7.40(m,3), 7.05(d,2), 6.95−6.75(m,7), 4.66(d,1), 4.52(d,1), 4.30−(m,1), 4.12(t,1), 3.78(m,1), 3.70(m,1), 3.62(m,1), 3.37(m,4), 3.22(m,4), 2.60(m,2), 2.09(q,1), 1.86(m,1), 1.73−(m,1), 1.40(d,3), 0.91(t,3). Mass spectrum (FAB): 669 ($M+H^+$).

EXAMPLE 24

(−)−[(5R)−cis]−4−[4−[4−[4−[[5−(2,4−Difluorophenyl)−5−(1H−1,2,4−triazol−1−ylmethyl)−tetrahydrofuran−3−yl]methoxy]phenyl]−1−piperazinyl]phenyl]−2,4−dihydro−2−[(1S)−(1−methylpropyl)]−3H−1,2,4−triazol−3−one

Follow the procedure of Example 23 except substitute an equivalent quantity of the product of Example 22.1 in place of the product of Example 21 to obtain the title compound, $[\alpha]_D^{25}$ − 22.2° (c=l, $CHCl_3$).

EXAMPLE 25

(+)−[(5S)−cis]−4−[4−[4−[4−[[5−(2,4−Difluorophenyl)−5−(1H−1,2,4−triazol−1−ylmethyl)−tetrahydrofuran−3−yl]methoxy]phenyl]−1−piperazinyl]phenyl]−2,4−dihydro−2−[(1S)−(1−methylpropyl)]−3H−1,2,4−triazol−3−one

Follow the procedure of Example 24 except substitute an equivalent quantity of the product of Example 16 in place of the product of Example 15 to obtain the title compound, $[\alpha]_D^{25}$ + 30.3° (c=l, $CHCl_3$); Calculated for $C_{36}H_{40}F_2N_8O_3$: C, 64.46; H, 6.01; N, 16.71; Found: C, 64.48; H, 5.96; N, 15.57.

EXAMPLE 26

(+)−[(5S)−cis]−4−[4−[4−[4−[[5−(2,4−Difluorophenyl)−5−(1H−1,2,4−triazol−1−ylmethyl)−tetrahydrofuran−3−yl]methoxy]phenyl]−1−piperazinyl]phenyl]−2,4−dihydro−2−[(1R)−(1−methylpropyl)]−3H−1,2,4−triazol−3−one

Follow the procedure of Example 23, except substitute an equivalent quantity of the product of Example 16 in place of the product of Example 15 to obtain the title compound, $[\alpha]_D^{25}$ + 22.4° (C=1, $CHCl_3$). Calculated for $C_{36}H_{40}F_2N_8O_3$: C, 64.46; H, 6.01 ; N, 16.71; Found: C, 64.47; H, 5.97; N, 16.56.

EXAMPLE 27

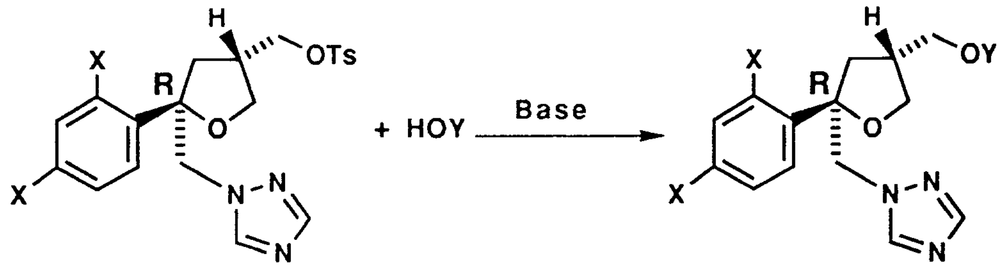


[ I ]

(R) – Series "cis – Tosylate" of Example 15

Follow the procedure of Example 23 except for the product of Example 21 substitute an equivalent quantity of one of the following six alcohols, HOY:

1.

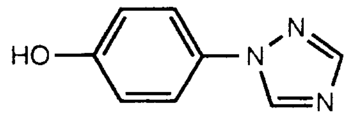

• Commercial Source: Aldrich 4 – (1H – 1,2,4 – Triazol – 1 – yl)phenol

2.

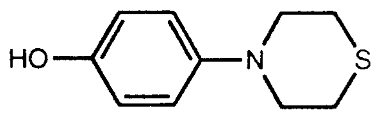

• Prepared according to the procedure of European Patent App. 0173258 4 – (Tetrahydro – 4H – 1,4 – thiazin – 4 – yl)phenol

3.

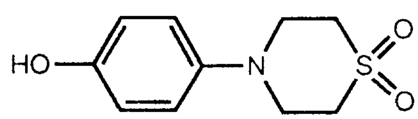

• Prepared according to the procedure of European Patent App. 0173258; 4 – (Tetrahydro – 4H – 1,4 – thiazin – 4 – yl – 4,4 – dioxide)phenol

4.

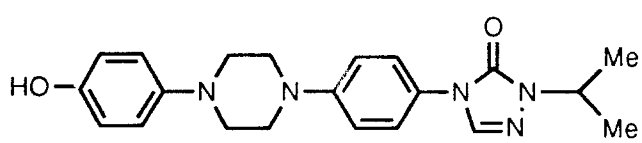

• See Example 22.2; 2,4 – Dihydro – 4 – [4 – (4 – hydroxyphenyl)] – 1 – piperazinyl] – phenyl – 2 – (1 – methylethyl) – 3H – 1,2,4 – triazol – 3 – one

5.

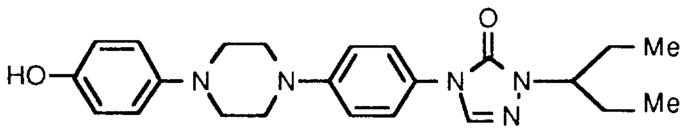

•See Example 22.3; 2,4 – Dihydro – 4 – [4 – (4 – hydroxyphenyl)] – 1 – piperazinyl] – phenyl] – 2 – (1 – ethylpropyl) – 3H – 1,2,4 – triazol – 3 – one

6.

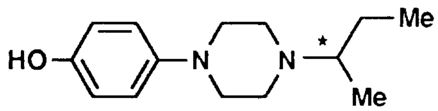

Prepared by reaction of:

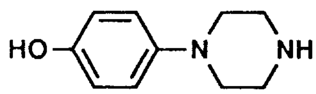

(available from Aldrich) with either R – ( – ) or S – ( + ) – 2 – Butanol tosylate according to Examples 19 to 22

7.

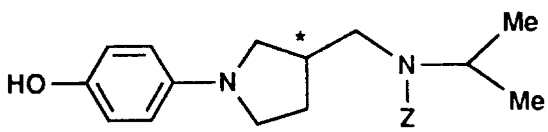

Examples 28 and 29

After the appropriate purification steps, there is produced a compound of formula [I] wherein X = F

Y =

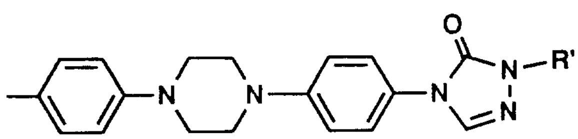

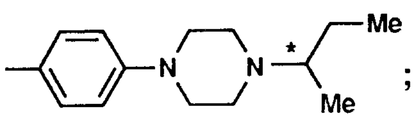 ; 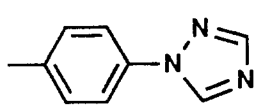 ;

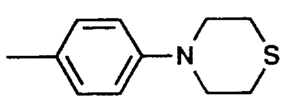 ; 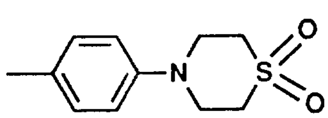 ; or

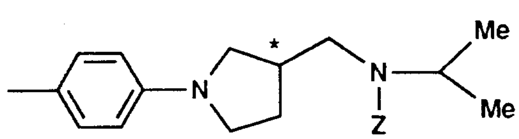

R' =

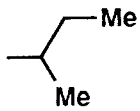 ; 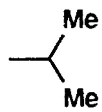 ; 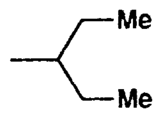 ;

and

Z = H, or ($C_1-C_5$) alkanoyl and the carbons with the asterisks(*) have the R or S absolute configuration.

Compounds of formula [I] wherein X = Cl may be prepared by use of the corresponding dichloro compound of Example 15. Compound wherein one X is F and the other is Cl may be prepared by use of the appropriate dihalophenyl compound.

EXAMPLE 28

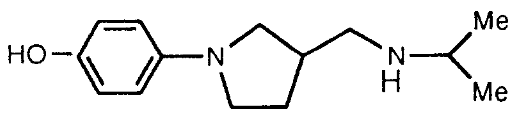

4-[3-(1-Methylethyl)amino]pyrrolidin-1-yl]phenol was prepared by the following Synthetic Scheme and Procedures A→C

Synthetic Scheme:

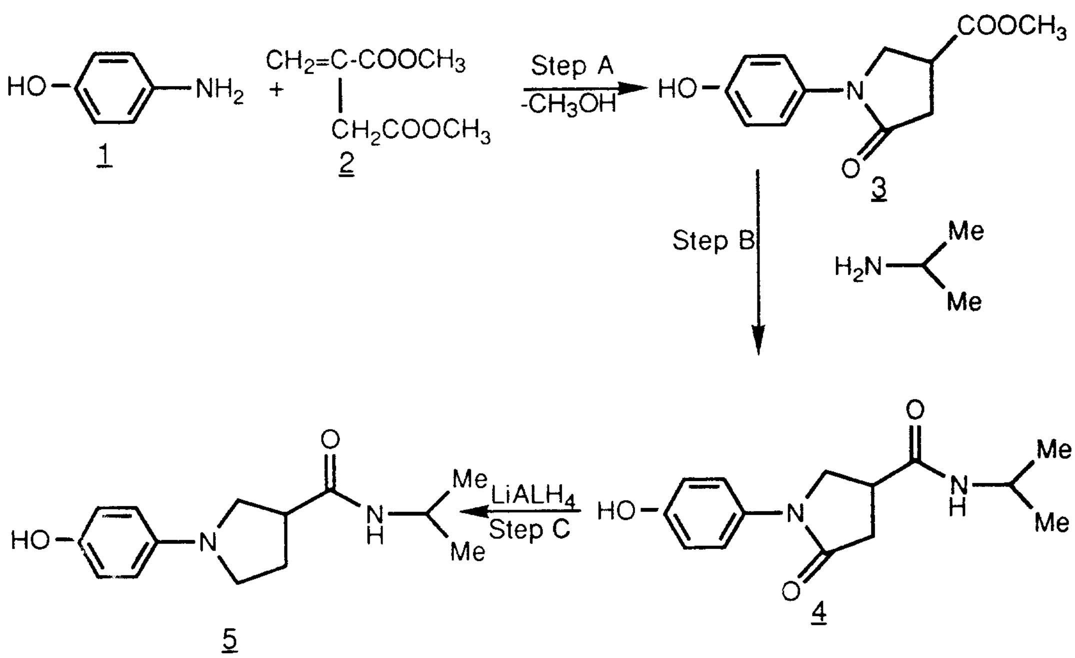


1 2 3 4 5

PROCEDURES

STEP A

A mixture of 4-aminophenol 1 (10.9g) and dimethyl itaconate 2 (15.8g) was heated (with stirring) at 180-195°C for 4 hours with continuous removal of methanol with a Dean Stark apparatus. The reaction mixture was cooled, dissolved in methanol (~50 ml) and poured into $CH_2Cl_2$ (1L). The organic solution was extracted with distilled water (~500 ml). An insoluble gum formed during the extraction, was removed by

decantation. The $CH_2Cl_2$ phase was dried over $MgSO_4$ (anhydrous) and evaporated in-vacuo to dryness to provide the crude product 3 (16.1g) which was purified by chromatography over silica gel using 1% $MeOH-CH_2Cl_2$ (v/v) as eluent. The progress of chromatography was followed by TLC using 5% $MeOH-CH_2Cl_2$ (v/v) as eluent. The pure fractions were combined and evaporated to dryness in-vacuo to provide 11.4g of pure 3. Molecular formula: $C_{12}H_{13}NO_4$ (M+ 235.7).

STEP B

A solution of 3 (5.8g) from Example 28A in methanol (100 mL) was treated with iso-propylamine (50 mL) and the so-formed mixture refluxed for 2 days. TLC of the reaction mixture showed the unchanged 3 still present; the reaction mixture was refluxed for 2 more days. The reaction mixture was evaporated to dryness in vacuo to provide crude 4 which was chromatographed on silica gel. Elution with 2% $MeOH-CH_2Cl_2$ (v/v) (containing conc. $NH_4OH$, 2mL per liter of solution) provided in some fractions, pure 4 - (4.98g). Mol. Formula: $C_{14}H_{18}N_2O_3$ ($M^+$ 262.2)

STEP C

A suspension of 4 (6.9g) from Example 28B in THF (150 mL; Aldrich Gold Label) was treated with stirring by dropwise addition of 1M $LiAlH_4$, (53.2 mL) over 10 minutes. After stirring at room temperature for 10 minutes, the reaction mixture was refluxed for ~12 hours. The so-formed reaction mixture is cooled and THF (250 mL) was added followed by dropwise addition of water (25 mL) over 10 minutes. The resulting suspension was removed by filtration through celite to form a filter cake which was washed with THF. The combined filtrates and washings were evaporated in-vacuo to dryness to provide crude 5 which was chromatographed on silica gel. The column was eluted with 1.5% $MeOH-CH_2Cl_2$ (v/v) (containing 1.5 mL concentration $NH_4OH$ per liter of solution) followed by 2.5% $MeOH-CH_2Cl_2$ (containing 2.5 mL concentration $NH_4OH$ per liter of solution). The fractions containing the desired product were combined and evaporated in-vacuo to dryness to provide 4.6g of pure 5. Molecular Formula: $C_{14}H_{22}N_2O$ ($M^+$234.3)

EXAMPLE 29

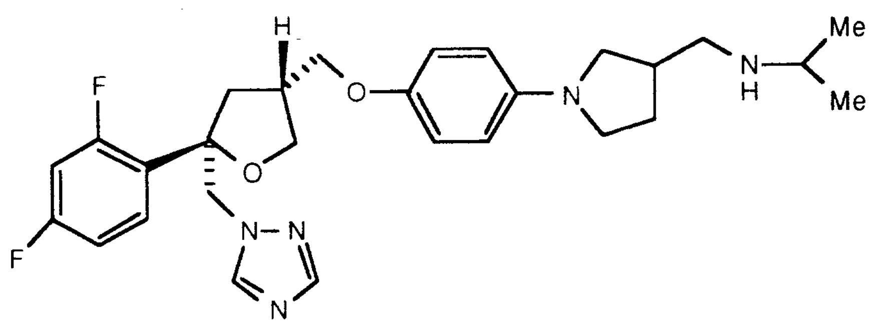

(5R-cis)-1-[4-[[5-(2,4-Difluorophenyl)tetrahydro-5-[(1-H-1,2,4-triazol-1-yl)methyl]-3-furanyl]methoxy]phenyl]-N-(1-methylethyl)-3-pyrrolidinamine

A solution of 4-[3-[(1-methylethyl)amino]pyrrolidin-1-yl]phenol of Example 28 (1.02g) in dry DMSO (20 mL; Aldrich Gold Label) was treated with sodium hydride (174 mgs) under argon atmosphere. The mixture was stirred at 50°C for 20 minutes followed by addition of a solution of the product of Example 15 (1.95g) in dry DMSO (20 mL). The so-formed reaction mixture was stirred at 80-90°C for 90 minutes, cooled and poured into EtoAc (500 mL). The organic phase was washed with water (500 mL) and brine (250 mL) the ethyl acetate solution was dried over anhydrous $MgSO_4$ and evaporated in-vacuo to dryness to provide crude title compound (2.34g) which was chromatographed over silica gel using 1% $MeOH-CH_2Cl_2$ (containing 1 mL of concentrated $NH_4OH$ per 1L solution) as eluent. Fractions containing the desired compound were combined and evaporated in-vacuo to dryness to provide pure title compound (1.6g). Molecular Formula $C_{26}H_{35}F_2N_5O_2$ ($M^+$511.6)

EXAMPLE 30

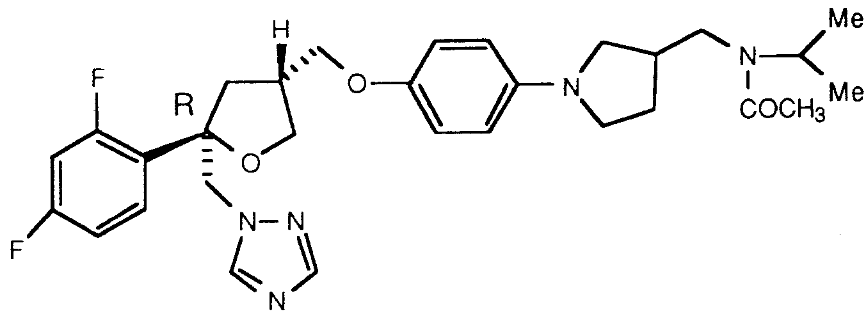

(5R-cis)-N-[1-[4-[[5-(2,4-Difluorophenyl)tetrahydro-5-[(1H-1,2,4-triazol-1-yl)methyl]-3-furanyl]methoxy]phenyl]-3-pyrrolidinyl]methyl]-N-(1-methylethyl)acetamide

A solution of the product from Example 29 (554 mg) in methanol (5 mL) was treated with acetic anhydride (under argon). The reaction mixture was stirred at room temperature overnight; methylene chloride (50 mL) was added followed by water (50 mL) and 10% $Na_2CO_3$ (5 mL). After stirring for ~5 minutes, the aqueous layer was separated and the $CH_2Cl_2$ layer was washed with water (50 mL). The $CH_2Cl_2$ phase was then dried over anhydrous $MgSO_4$ and evaporated in-vacuo to dryness to provide the crude title compound (550 mgs). Chromatography of the crude product over silica gel using 1% MeOH-$CH_2Cl_2$ (containing 1 mL of concentrated $NH_4OH$ per 1L of solution as eluant) provides in some fractions, pure title compound (340 mg); Molecular Formula $C_{30}H_{37}F_2N_5O_3$; ($M^+$553.6).

COMPARATIVE EXAMPLE 31

The in vivo oral antifungal activity of the compounds of Example 23-26 were compared to those of itraconazole, fluconazole and saperconazole in an Aspergillus pulmonary infection model in mice. The procedure of David Loebenberg et al. entitled "Sch 42427, The Active Enantiomer of Antifungal agent Sch 39304: In Vitro and In Vivo Activity" Antimicrobial Agents and Chemotherapy (1992), 36 498-501 was used. The Aspergillus flavus pulmonary model was run in the following manner Male, CF-1 mice weighing 20 grams were compromised with cortisone acetate (100 mg/kg), administered subcutaneously, once daily for 3 days. In addition, to prevent bacterial disease, tetracycline HCL (300 mg/1) was added to the drinking water and given ad libitum. On day 2 of compromising, mice were infected in an inhalation chamber, first described by Piggot and Emmons in 1960 and modified by us. The chamber is a 1 liter pyrex, thick-walled flask with 8 tubular side-arms that extend into the flask. Each side-arm is a pyrex tube of 14 cm length, constricted to a 1 cm opening inside the flask. The bottom of the flask was covered with a malt extract agar medium on which a sporulating culture of A. flavus ATCC 24133 was grown for 13 days at room temperature. The top of the flask was closed with a #10 stopper through which passed a glass tube attached by rubber tubing to a 60 mL syringe. Mice were placed in each of the side arms and pushed to the bottoms so that their nares extended beyond the open end of the tube and over the agar. Cotton plugs were then inserted behind the mice to hold them in place. By pumping the 60 mL syringe twice, air was forced over the culture and produced a cloud of spores. Mice were exposed to the spore cloud for one minute. Within 15-30 minutes after exposure, a number of mice were sacrificed and lung tissue samples homogenized for culture to determine the number of inhaled conidia. Oral treatment began 24 h post-infection with doses of 5 to 250 mg of drug/kg in ethanol; vehicle (115 ml of Emulphor EL-719P, GAF Wayne, NJ; and 5 ml of 20% w/v lactic acid per liter of water), 10:90 v/v, once daily for 4 days.

The compounds of this invention of formula IIa (Example 23) and II (b) (Example 24) were more active orally in the Aspergillus pulmonary model than itraconazole, saperconazole and fluconazole. The results are graphically displayed in Figure 1 for 100 mg of drug per kg of body weight of compromised mice infected by inhalation of Aspergillus flavus spores.

TABLE I

IN VIVO ORAL ANTIFUNGAL ACTIVITY
ASPERGILLUS LUNG INFECTION IN MICE
ANIMALS TREATED EITHER ONCE
OR 3 TIMES A DAY FOR 4 DAYS

RESULTS 18 DAYS POST INFECTION

| COMPOUNDS | DOSE(mg/kg) | %SURVIVAL | %ANIMALS NEG[a] | CFU-GEO MEAN OF SURVIVORS[b] |
|---|---|---|---|---|
| Example 26 | 100 | 20 | 0 | ND[c] |
| - | 66 | 0 | 0 | - |
| - | 33 | 0 | 0 | - |
| - | 33 [3RX/DAY] | 0 | 0 | - |
| Example 25 | 100 | 10 | 0 | ND |
| - | 66 | 0 | 0 | - |
| - | 33 | 0 | 0 | - |
| - | 33 [3RX/DAY] | 20 | 10 | ND |
| Example 23 | 100 | 70 | 10 | 1.85 |
| (Formula IIa) - | 66 | 10 | 0 | ND |
| - | 33 | 10 | 0 | ND |
| - | 33 [3RX/DAY] | 60 | 10 | 1.08 |
| Example 24 | 100 | 100 | 20 | 1.95 |
| (Formula IIb) - | 66 | 50 | 0 | 2.25 |
| - | 33 | 0 | 0 | - |
| - | 33 [3RX/DAY] | 40 | 0 | 2.4 |
| Itraconazole | 100 | 20 | 0 | ND |
| - | 66 | 0 | 0 | - |
| - | 33 | 0 | 0 | - |
| - | 33 [3RX/DAY] | 10 | 0 | ND |
| Fluconazole | 100 | 10 | 0 | ND |
| Saperconazole | 100 | 0 | 0 | - |

a - <10 colonies
b- CFU-Geo mean of survivors - Geometric mean of the logarithm of the Colony Forming Units remaining in the lung of the mice
c - Not done

EXAMPLE 32

(2RS)-(±)cis-1-[4-[[2-(2,4-Difluorophenyl)-2-[(1H,1,2,4-triazol-1-yl-methyl]tetrahydro-4-furanyl]methoxy]phenyl]-4-(1-methylethyl)piperazine prepared in accordance with Example 68 of PCT/US88/03987 and USP 5,039,676 as well as the R-(-) and S(+) enantiomers thereof were tested for antifungal activity. The R(-) and S(+) enantiomers were separated by use of preparative HPLC on a Chiralcel (5x50 in ID) preparative column equilibrated with 70:30 (v/v) hexane: ethanol; elution was done with

70:30 to 50:50 v/v hexane: ethanol. The R(−) enantiomer of the compound of Example 68 was more active orally in a mouse Candida infection model performed in accordance with the procedure of described hereinbelow in Example 33 than the S−(+) enantiomer. The R(−) enantiomer of this Example was found inactive in the in vivo oral mouse Aspergillus lung infection described in Example 31.

COMPARATIVE EXAMPLE 33

The compounds of Examples 23−26 and itraconazole, fluconazole, and saperconazole were tested for in vivo oral antifungal activity in a Candida systemic model using CF1 male mice , average weight 20 g, Harlan Sprague Dawley, Inc., Indianapolis, Ind. infected by IV injection into the tail vein of C. albicans C−43 (5 million CFUs). The drugs were dissolved in polyethylene glycol−200 (PEG−200) and tested by orally administering 50, 25 and 10 mpk of each drug 4 hours post infection and once daily for 3 more days. Oral efficacy was measured after four (4) days by percent survival and by the number of organisms remaining in the kidneys i.e., the Colony Forming Units (CFUs). The mice were sacrificed and the kidneys of individual mice were homogenized in sterile saline, diluted and spread onto Mycosel agar. Colony counts were determined after 48 hours at 37°C. For calculation of geometric means, mice that died during the experiment were considered to have $10^9$ CFUs/kidneys (based on numerous previous experiments). The preferred compounds of this invention of Example 23 (formula II a) and Example 24 (IIb) were more active orally in this model than itraconazole at doses of 50, 25 and 10 mpk in (each dissolved in PEG−200). The results are summarized in Table II

TABLE II

IN VIVO ORAL ANTIFUNGAL ACTIVITY AGAINST SYSTEMIC CANDIDIASIS

CF1 Mice Infected with C. albicans C43 (5 million CFUs)

| Antifungal Compound | Vehicle | MPK (PO) | Results (day 4 post-infection) % Survival | CFUs (GM)[1] |
|---|---|---|---|---|
| Example 26 | PEG-200 | 50 | 20 | 7.84 |
| | | 25 | 0 | 9.00 |
| | | 10 | 0 | 9.00 |
| Example 25 | PEG-200 | 50 | 0 | 9.00 |
| | | 25 | 0 | 9.00 |
| | | 10 | 0 | 9.00 |
| Example 23 (II a) | PEG-200 | 50 | 90 | 5.62 |
| | | 25 | 80 | 5.90 |
| | | 10 | 50 | 6.78 |
| | | 1 | 10 | 8.57 |
| Example 24 (II b) | PEG-200 | 50 | 100 | 4.99 |
| | | 25 | 100 | 5.14 |
| | | 10 | 40 | 7.42 |
| | | 1 | 30 | 7.88 |
| Itraconazole | PEG-200 | 50 | 60 | 6.68 |
| | | 25 | 0 | 9.00 |
| | | 10 | 0 | 9.00 |
| Fluconazole | ETOH/Mon | 10 | 90 | 5.68 |
| | | 1 | 70 | 6.58 |
| Saperconazole | PEG-200 | 50 | 20 | 8.20 |
| None | ETOH/Mon | - | 0 | 9.00 |
| " | PEG-200 | - | 0 | 9.00 |
| " | - | - | 0 | 9.00 |

Footnotes

Treatment: 1 a day x 4 days
Vehicles: PEG-200-polyethylene glycol 200
ETOH/Mon - 10% ethanol/90% vehicle (See Comparative Example 31)

1. CFUs (GM-) - The geometric mean of the logarithims of the Colony Forming Units remaining in the kidneys of the mice.

COMPARATIVE EXAMPLE 34

The procedure of Example 31 was used to compare the in vivo oral antifungal activity of (±)-5R/S-(cis)-4-[4-[4-[4-[[5-(2,4-difluorophenyl)-5-(1H-1,2,4-triazol-1-ylmethyl) tetrahydrofuran-3-yl]methoxy-phenyl-1-piperazinyl]phenyl]-2,4-dihydro-2[(R/S)-(1-methylpropyl)]-3H-1,2,4-triazol-3-one with (±)-2R/S-(cis)-1-[4-[[2-(2,4-difluorophenyl)-2-(1H-1,2,4-triazol-1-yl-methyl) tetrahydro-4-furanyl]methoxy]phenyl]-4-(1-methylethyl)piperazine of Example 68 of USP 5,039,676 in an Aspergillus pulmonary infection model described in Example 31. As shown in Table III, the

compound of Example 68 of US Patent 5,039,676 was inactive in this animal model.

TABLE III

IN VIVO ORAL[1] ACTIVITY AGAINST AN ASPERGILLUS FLAVUS PLUMONARY INFECTION IN MICE

| Compound[2] | Dose mg/kg | Percent Survival After 11 Days |
|---|---|---|
| 1. (±)-5R/S-(cis)-4-[4-[4-[4-[[5-(2,4-difluorophenyl)-5-(1H-1,2,4-triazol-1-ylmethyl)tetrahydrofuran-3-yl]methoxyphenyl-1-piperazinyl]phenyl]-2,4-dihydro-2[(RS)-(1-methylpropyl)]-3H-1,2,4-triazol-3-one[3] | 200<br>100<br>50 | 50.0<br>41.7<br>16.7 |
| 2. (±)-R/S-cis | 200 | 0 |
| Compound of | 100 | 0 |
| Example 68 of W 89/04824 and USP 5,039,676 | 50 | 0 |

1 OD for 4 Days
2 Compounds dissolved in PEG-200 were administered (PO) for 4 days to CF-1 mice infected with Aspergillus flavus.
3 The (±)-5R/S(cis)5-(2,4,-difluorophenyl)tetrahydro-5-[1H-1,2,4-triazol-1-yl)methyl]-3-furanmethanol prepared in accordance with the procedure of Example 68(c) of USP 5,039,676 was converted into the (±)-(5R/S)-cis tosylate by standard conditions (tosyl chloride and pyridine) followed by chromatography as described in Example 15.

(±) - 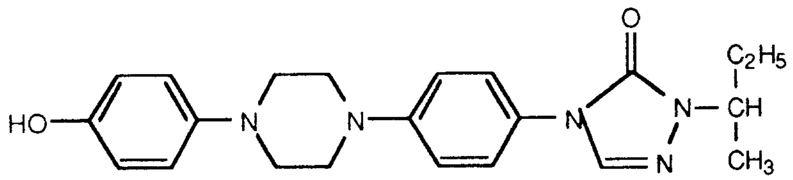

(prepared in accordance with the procedure of J. Heeres et al. J. Med Chem (1984), 27, 894-900) in the presence of NaH in dry DMSO at 50°C for 30 min. The reaction mixture was stirred at 80° - 90°C for 1 hr and poured into $CH_2Cl_2$ and EtOAc and brine. The organic layers were separated, washed with water and brine and dried over $MgSO_4$. The solvent was evaported to give a crude product which was purified by silica gel chromatograph to give (±) 5RS (cis)-4-[4-[4-[4-[[5-(2,4-difluorophenyl)-5-(1H-1,2,4-triazol-1-ylmethyl) tetrahydrofuran3-yl]methoxy]phenyl]-1-piperazinyl]-2,4-dihydro-2[(RS)-(1-methylpropyl)]-3H-1,2,4-triazol-3-one.

## COMPARATIVE EXAMPLE 35

The procedures of Examples 31 and 34 were used.

TABLE IV

IN VIVO ORAL ACTIVITY (mg/kg) AGAINST AN ASPERGILLUS PULMONARY INFECTION IN MICE

PERCENT SURVIVAL AFTER 5 DAYS (5D) AND 10 DAYS (10D)

| Dose (mg/kg)[1]: | 50 | | 100 | | 200 | |
|---|---|---|---|---|---|---|
| Compound 1 of Table III of Examples 34[2] | 5D | 10D | 5D | 10D | 5D | 10D |
| | 42 | 17 | 50 | 42 | 50 | 50 |
| Saperconazole | 0 | 0 | 50 | 33 | 25 | 0 |
| Itraconazole | 8 | 0 | 17 | 0 | 25 | 17 |
| PEG-200 | 8 | 0 | | | | |

1 Compounds dissolved in PEG-200 were administered daily for 4 days to CF-1 mice infected with Aspergillus flavus spores.

2 Prepared as described in Example 34, footnote 3

COMPARATIVE EXAMPLE 36

The compounds of Examples 23-24 and itraconazole, fluconazole, were tested for in vivo oral antifungal anctivity in a Candida systemic model using normal and compromised CF1 mice infected with C. albicans C-65 (5 million CFUs). The procedure of Example 33 was followed. The drugs were dissolved in polyethylene glycol-200 ("PEG-200) at room temperature and tested by orally administering 100, 50, 25, 10 and 1 mpk of each drug. Oral efficacy was measured by percent survival and by the number of organisms remaining in the kidneys (CFUs) after four days. The preferred compounds of this invention of Example 23 (formula IIa) and Example 24 (IIb) were more active orally in this model than itraconazole at doses of 50 and 25 mpk; each drug was dissolved in PEG-200. The pharmaceutical composition of the preferred compound of formula IIb of Eample 24 in hydroxypropyl beta cyclodextrin ("HPβCD") having 7.4 hydroxypropyl groups per HPβCD (obtained from Pharmatec, Inc., Alachua, FL 32615) was prepared by admixing the appropriate amount of the compound IIa with a 40% (w/v) solution of HPβCD (4 g of HPβCD per 10 mL of purified water). Gentle heating was used to form a clear solution. For the 10 mpk dose, 12 mg of IIa was added to 6 mL of a 40% w/v solution of HPβCD. For dilutions, sterile water was added to the solution with mixing. The pharmaceutical composition of itraconazole and the Pharmatec HPβCD described above was prepared as follows. Propylene glycol (10 mL) was admixed with 0.95 mL of concentrated HCl at 40°-45°C with stirring. Itraconazole (2.5 g) was added thereto and stirring was continued until homogeneous. The mixture so formed was cooled to 20° to 30°C and admixed with a solution of 60 g of HPβCD in 40 mL of purified water to form a clear solution. The pH was adjusted to a value of 1.9-2.1 with 10N NaOH and sufficient purified water added (with mixing) to a final volume of 100 mL. For dilutions, sterile water was added to the itraconazole - HPβCD. The pharmaceutical composition of the compound of formula IIb and HPβCD was more active orally in the Candida systemic model in normal mice at 1 mpk and in compromised mice at 10 mpk than the pharmaceutical composition of itraconazole and HPβCD. The results are summarized in Table V.

TABLE V

IN-VIVO ORAL ANTIFUNGAL ACTIVITY AGAINST A SYSTEMIC CANDIDIA ALBICANS C65 (5 MILLION CFUs) INFECTION IN NORMAL AND COMPROMISED CF-1 MICE.

Results (day 4 post-infection)

| | | | Normal Mice[1] | | 600 Rads Mice[2] | |
|---|---|---|---|---|---|---|
| Compound | Vehicle | MPK (PO) | % S[3] | CFUs (GM)[4] | %S | CFUs (GM) |
| Example 23 | PEG-200 | 100 | ND[5] | ND | 50 | 6.43 |
| | | 50 | 90 | 4.69 | 30 | 7.33 |
| | | 25 | 60 | 5.74 | 20 | 8.65 |
| | | 10 | 30 | 7.12 | 0 | 9.00 |
| | | 1 | 0 | 9.00 | ND | ND |
| Example 24 | PEG-200 | 100 | ND | ND | 80 | 6.28 |
| | | 50 | 100 | 4.44 | 30 | 7.58 |
| | | 25 | 90 | 4.67 | 20 | 8.27 |
| | | 10 | 40 | 7.48 | 0 | 9.00 |
| | | 1 | 30 | 7.73 | ND | ND |
| Example 24 | Beta-CD | 10 | 100 | 4.94 | 30 | 7.88 |
| | | 1 | 70 | 5.97 | ND | ND |
| Itraconazole | PEG-200 | 100 | ND | ND | 20 | 8.45 |
| | | 50 | 60 | 5.98 | 0 | 9.00 |
| | | 25 | 50 | 6.93 | 0 | 9.00 |
| | | 10 | 30 | 7.64 | 0 | 9.00 |

| Cont. | | | Normal Mice[1] | | 600 Rads Mice[2] | |
|---|---|---|---|---|---|---|
| Compound | Vehicle | MPK (PO) | % S[3] | CFUs (GM)[4] | %S | CFUs (GM) |
| | | 1 | 0 | 900 | ND | ND |
| Itraconazole | Beta-CD-H | 25 | 100 | 6.19 | 60 | 7.98 |
| | | 10 | 0 | 9.00 | 30 | 8.11 |
| Fluconazole | PEG-200 | 25 | ND | ND | 50 | 6.91 |
| | | 10 | 80 | 5.35 | 20 | 8.01 |
| | | 1 | 60 | 5.81 | ND | ND |
| - | PEG-200 | - | 20 | 8.14 | 0 | 9.00 |
| - | Beta-CD[6] | - | 0 | 9.00 | 0 | 9.00 |
| - | Beta-CH[7] | - | 20 | 8.48 | 0 | 9.00 |
| - | - | - | 0 | 9.00 | 0 | 9.00 |

Treatment 1/day x 4 days ND: not done

Vehicles PEG-200: polyethylene glycol 200

Beta-CD: hydroyproply-Beta-cyclodextrin having 7.42 HP groups per HPβCD

1 CF-1 mice white, male, average weight 20 g, Harlan, Sprague Dawley, Inc. Indianapolis, Ind.

2 CF-1 mice compromised with 600 Rads of gamma irradiation.

3. %S is Percent Survival.

4 CFUs (GM)-Geometric mean of the logarithims of the Colony Forming Units in the Kidneys of the mice determined as described in Example 33.

5. ND - Not Done.

6. Hydroxylpropyl-β-cyclodextrin vehicle used for pharmaceutical composition of the compound of Example 24 reported in Table V.

7. Hydroxypropyl-β-cyclodextrin vehicle used for the pharmaceutical composition of itraconazole reported in Table V.

EXAMPLE: 37

[2R,4R]-4-(2,4-Difluorophenyl)-2-hydroxymethyl-5-[1H-1,2,4-triazol-l-yl)]-1,4-pentanediol-1-acetate

Combine 2g of the product of Example 11 and 5g of porcine pancreatic lipase (Sigma Chemical Co., L3126) in 100 mL of EtOAc. Stir the mixture at ambient temperature for 24 hrs, and filter the mixture. Evaporate the solvent and chromatograph the residue on silica gel, eluting with 9:1 EtOAc-acetone to give 1.1g of the title compound: PMR($CDCl_3$) δ 7.94 (s, 1), 7.80 (s, 1), 7.48 (m, 1), 6.78 (m, 2), 4.72 (d, 1), 4.3 (d, 1), 4.12 (m, 2), 3.39 (m, 2), 2.2-1.8 (m, 6).

EXAMPLE: 38

[2R,4R]-4-(2,4-Difluorophenyl)-2-[(2-tetrahydropyranyl)oxymethyl]-5-[1H-(1,2,4-triazol-l-yl)]-l,4-pentanediol-1-acetate.

Dissolve 1g of the product of Example 37 in 30 mL of $CH_2Cl_2$, add 5 mL of dihydropyran and 0.7g of pyridinium p-toluene sulfonate, and stir the solution for 18 hrs. Wash the solution with water, dry the organic layer over anhydrous Mg $SO_4$, and filter the mixture. Evaporate the filtrate and chromatograph the residue on silica gel. Elute with 9:1 EtOAc-acetone to give 0.9g of the title compound.
PMR ($CDCl_3$ $\delta$ 8.03 and 8.01 (2 X s, 1), 7.83 (s, 1), 7.5 (mc 1), 6.8 (m, 2), 4.41 (d, 1), 4.55-4.30 (m, 2), 4.12 (m, 2), 3.9-3.4 (m, 3), 3.11 (m, 1) 2.3-1.4 (m, 12).

EXAMPLE: 39

[2S,4R]-4-(2,4-Difluorophenyl)-2-[(2-tetrahydropyranyl)oxymethyl]-5-[1H-(1,2,4-triazol-l-yl]-1,4-pentanediol

Combine 0.9g of the product of Example 38, 60 mL of THF, and 20 mL of IN aqueous KOH. Stir the mixture for 18 hrs, pour it into $Et_2O$, and dry the organic layer over anhydrous $MgSO_4$. Filter the mixture, evaporate the filtrate, and chromatograph the residue on silica gel. Elute with 95:5 EtOAc-acetone to give 0.5g of the title compound: PMR ($CDCl_3$) $\delta$ 8.12 and 8.10 (2 X s 1), 7.89 9s, 1) 7.55 (m, 1), 6.8 (m, 2), 4.54 (s, 2), 4.43 (m, 1), 3.7 (m, 2), 3.5 (m, 3), 3.15 (m, 1), 2.4 (m, 1), 1.9-1.4 (m, 8).

EXAMPLE: 40

[2R,4R]-4-(2,4-Difluorophenyl)-2[(2-tetrahydropyranyl) oxymethyl]-5-[1H-(1,2,4-triazol-1-yl]-1,4-pentanediol, 1-[(4-methylphenyl) sulfonate

Dissolve 0.5g of the product of Example 39 in 20 mL of THF. Add 0.05g of N,N-dimethylamino pyridine, 0.3 mL of $Et_3N$, and then 0.26g of [(4-methylphenyl)sulfonyl] chloride. Stir the mixture at ambient temperature for 18 hrs, and then filter the mixture. Evaporate the solution and chromatograph the residue on silica gel. Elute with 95:5 EtOAc-acetone to give 0.55g of the title compound (which was used in the following step without further purification or characterization).

EXAMPLE: 41

(-)-(5R-(cis)-5-(2,4-Difluorophenyl)-5-[(1H-1,2,4-triazol-l-yl)methyl]tetrahydro-3-furanmethanol

Dissolve 0.55g of the product of Example 40 in 20 mL of THF and add 80 mg of 60% NaH dispersion in oil. Stir the mixture at ambient temperature for 1 hr, and then pour it into water. Extract the mixture with EtOAc, dry the extract over anhydrous $MgSO_4$, filter the mixture, and evaporate the filtrate.

Dissolve the residue in 20 mL of MeOH, add 100 mg of (4-methylphenyl)-sulfonic acid, and stir the solution at ambient temperature for 18 hrs. Add 1 mL of $NH_4OH$, concentrate the solution, and partition to residue with EtOAc-$H_2O$. Dry the organic solvent over anhydrous $MgSO_4$, filter the mixture, and evaporate the filtrate. Chromatograph the residue on silica gel. Elute with 8:2 EtOAc-acetone to give 0.5g of the title compound: PMR ($CDCl_3$) d 8.11 (s, 1), 7.81 (s, 1), 7.35 (m, 1), 6.81 (m,2), 4.57 (q. 2), 4.04 (t, 1), 3.72 (m, 1); 3.4 (m, 2), 2.55-2.25 (m, 2), 2.05-1.90 (m, 1).

To verify the stereochemistry, react the title compound with [(4-methylphenyl)sulfonyl chloride and pyridine following standard procedure to give a product identical in all respects to the product of Example 15.

EXAMPLE: 42

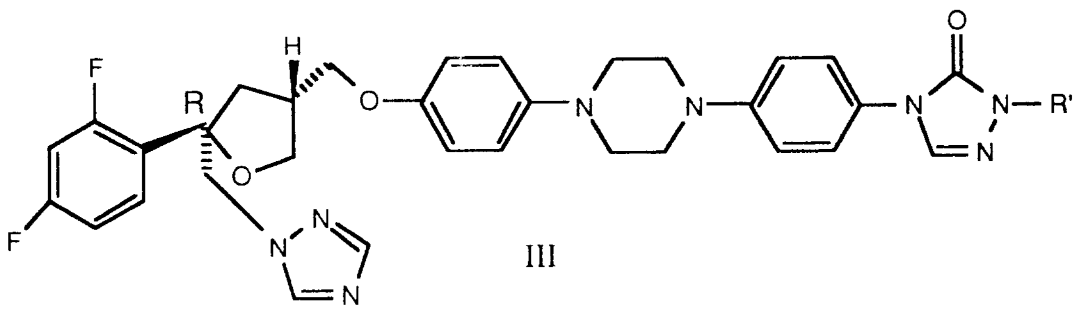

III

A.

Preparation of

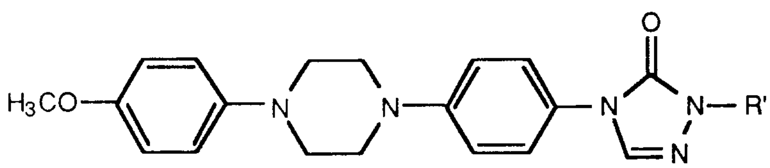

IV

Follow the procedure of Examples 19 and 20 except substitute an equivalent quantity of a compound, in Column A below for the S-(+)-2-butanol tosylate of Example 19 to obtain a prduct of the formula IV wherein R' is as shown in Column B.

| Example | Column A | Column B<br>R' |
|---|---|---|
| 42a | 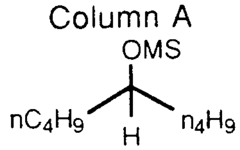 | $-CH(\underline{n}C_4H_9)_2$ |
| 42b | $MSOCH_2CF$ | $-CH_2CF_3$ |
| 42c | $Cl(CH_2)_2N(CH_3)_2$ | $-(CH_2)_2N(CH_3)_2$ |
| 42d | $TsO(CH_2)_3C\equiv CH$ | $-(CH_2)_3C\equiv C-H$ |
| 42e | $MsOCH_2CH=CHC_2H_5$ | $-CH_2CH=CHC_2H_5$ |
| 42f | $MsO^*CH(C_2H_5)(C_4H_9)$ | $-^*CH(C_2H_5)(C_4H_9)$ |
| 42g | $MsO^*CH(C_2H_5)(CH_2C\equiv CH)$ | $-^*CH(C_2H_5)(CH_2C\equiv CH)$ |
| 42h | $MsOCH_2C\equiv CCH_3$ | $-CH_2C\equiv CCH_3$ |
| 42i | $MsO^*CH(CH_3)CH_2CH=CH_2$ | $-^*CH(CH_3)CH_2CH=CH_2$ |
| 42j | $MsO(CH_2)_2CH=C(CH_3)_2$ | $-(CH_2)_2CH=C(CH_3)_2$ |
| 42k | $ClCH_2CO_2CH_3$ | $-CH_2CO_2CH_3$ |
| 42l | $MsOCH_2CH=CHC\equiv C(CH_3)_3$ | $-CH_2-CH=CHC\equiv C(CH_3)_3$ |
| 42m | $MsOCH_2C\equiv C-C\equiv C(CH_3)_3$ | $-CH_2-C\equiv C-C\equiv C(CH_3)_3$ |

B.

Follow the procedures of Examples 21 and 23 except substitute an equivalent quantity of a compound of Part A of Example 42 for the compound of Example 21 to obtain the corresponding demethylated products then substitute an equivalent quantity of the demethylated products for the demethylated product in Example 23 to obtain the compounds of formula III where R' is as shown in Column B of Step A.

## Claims

1. A compound represented by formula [I]

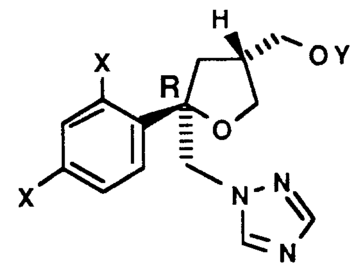

[ I ]

wherein X is independently both F or both Cl or one X is independently F and the other is independently Cl;

Y =

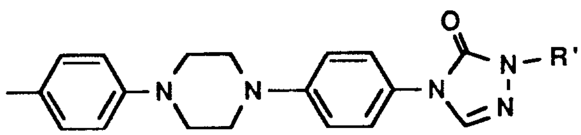 ; 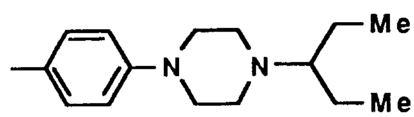 ;

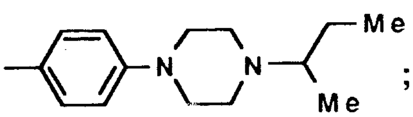 ; 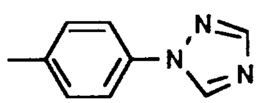 ;

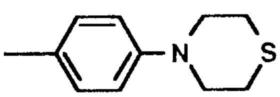 ; 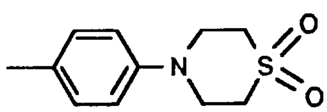 ; or

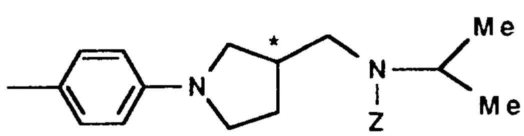

R' = $(C_1-C_{10})$ alkyl; $(C_2-C_{10})$alkenyl; $(C_2-C_{10})$alkynyl; $(C_3-C_8)$cycloalky or $CH_2R^2$;
$R^2$ = $(C_1-C_3)$perhaloalkyl; $CO_2R^3$, *$CH(OR^4)CH_2OR^4$ or $CH_2N(R^5)$
$R^3$ = lower alkyl or H
$R^4$ = $R^3$ or $(CH_2)_2OR^3$
$R^5$ = lower alkyl

Z = H, or $(C_1-C_5)$ alkanoyl and the carbons with the asterisks (*) have the R or S absolute configuration; or a pharmaceutically acceptable salt thereof

2. A compound represented by the formula Ia

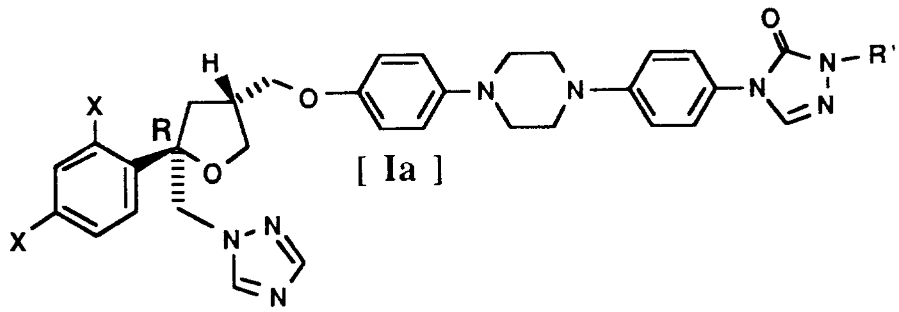

[ Ia ]

wherein X is independently both F or both Cl or one X is independently F and the other is independently Cl;

R' =

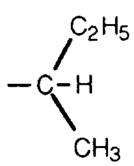 , 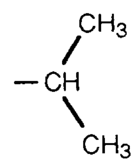 , 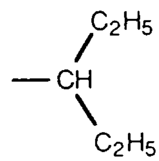 ,

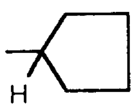 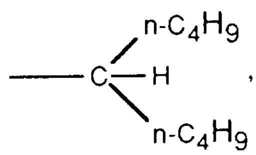 , 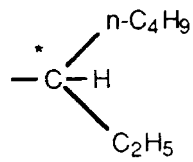 ,

$—CH_2CO_2H$, $-CH_2CH_2N(CH_3)_2$, $-(CH_2)_4C{\equiv}CH$

$-CH_2CH{=}CHC_2H_5$, 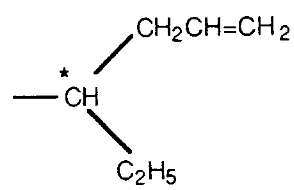 ,

$-CH_2-C{\equiv}C-CH_3$, 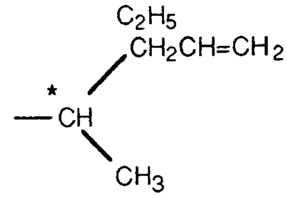 ,

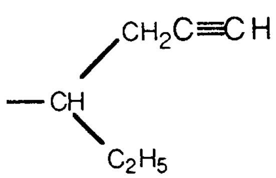 , 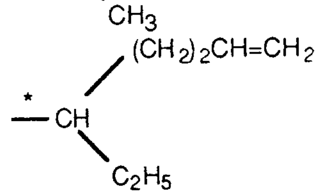 ,

$-(CH_2)CH{=}C(CH_3)_2$ $-CH_2CH{=}CHCH(CH_3)_3$ and

$-CH_2-{}^{*}CH(OH)CH_2OH$

and the carbons with the asterisk (*) have the R or S absolute configuration; or a pharmaceutically acceptable salt thereof.

**3.** A compound represented by the formula II

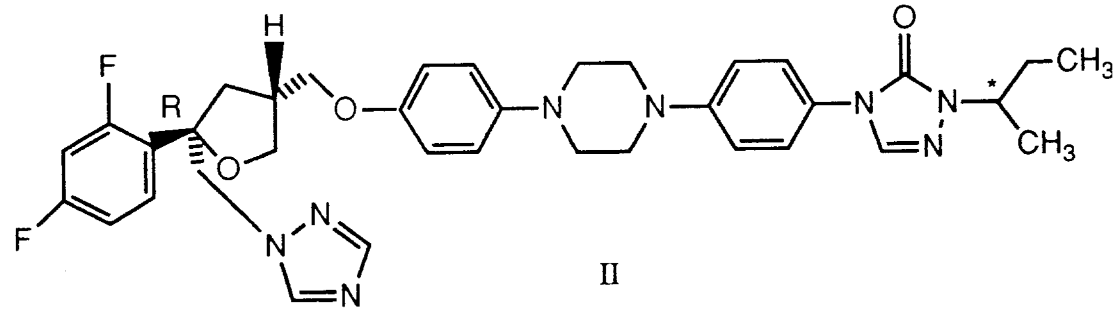

II

wherein the carbon with the asterisk has the R or S absolute configuration and stereochemical isomers thereof or a pharmaceutically acceptable salt thereof

**4.** A compound of claim 3 which is (−)−[(5R)−cis]−4−[4−[4−[4−[[5−(2,4−difluorophenyl)−5−(1H−1,2,4−triazol−1−ylmethyl) tetrahydrofuran−3−yl]methoxy)−phenyl−1−piperazinyl]phenyl]−2,4−dihydro−2[(R)−(1−methylpropyl)]−3H−1,2,4−triazol−3−one and is represented by the formula IIa;

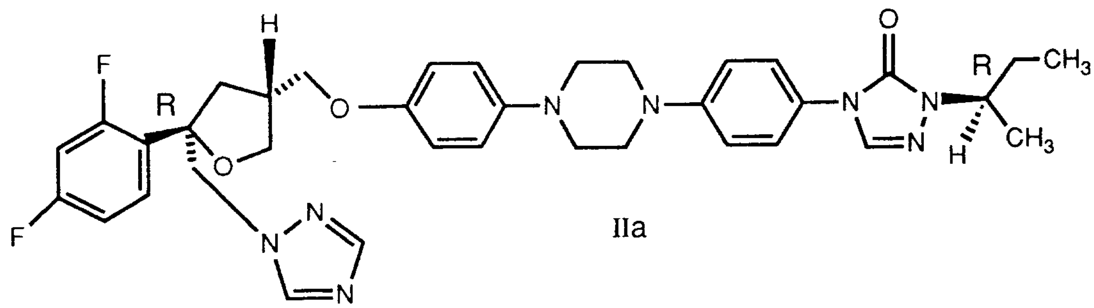

IIa

or which is (−)−[(5R)−cis]−4−[4−[4−[4−[[5−(2,4−difluorophenyl)−5−(1H−1,2,4−triazol−1−ylmethyl) tetrahydrofuran−3−yl)methoxy]−phenyl−1−piperazinyl]phenyl]−2,4−dihydro−2[(S)−(1−methylpropyl)]−3H−1,2,4−triazol−3−one and is represented by the formula IIb;

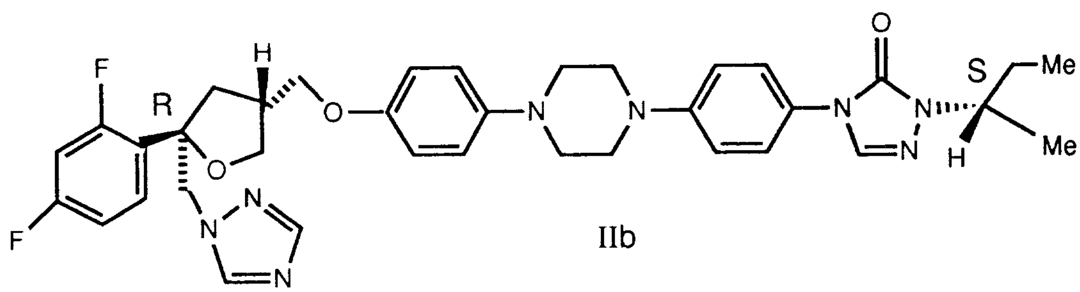

IIb

or which is named (−)−[(5R)−*cis*−[−4−[4−[4−[4−[[5−(2,4−difluorophenyl)−5−(1H−1,2,40−triazol−1−ylmethyl)tetrahydrofuran−3−yl]methoxy]phenyl]−1−piperazinyl]phenyl)−2, 4−dihydro−2−(3−pentyl)]−3H−1,2,4−triazol−3−one and is represented by formula IIc.

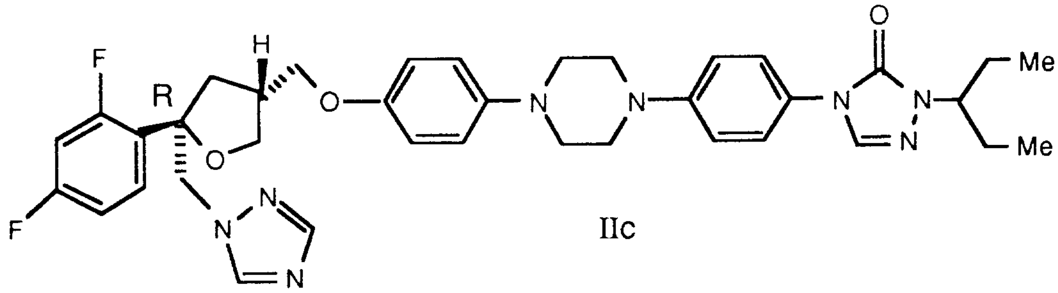

IIc

5. A pharmaceutical composition for treating fungal infections comprising an antifungally effective amount of a compound of claim 1 and a pharmaceutically acceptable carrier therefor.

6. The pharmaceutical composition of claim 5 wherein the carrier is a hydroxypropyl-$\alpha$-, $\beta$- or $\gamma$-cyclodextrin.

7. The pharmaceutical composition of claim 5 which further comprises hydroxypropyl-$\beta$-cyclodextrin having about 2 to about 11 hydroxypropyl groups per molecule.

8. The pharmaceutical composition of claim 5 which comprises a fungicidally effective amount of a cell wall active compound.

9. A method of treating or preventing fungal infections in mammals in need of such treating or preventing such infections which comprises administering to such a mammal an antifungally effective amount of a compound of claim 1.

10. A compound represented by the formulas III, IV, V or VI

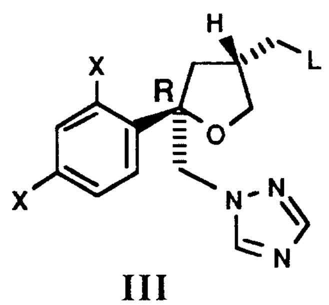

III

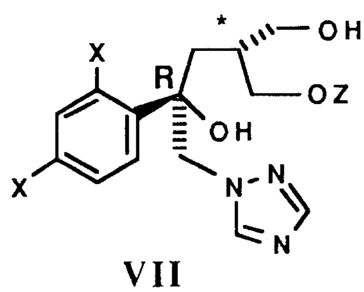

VII

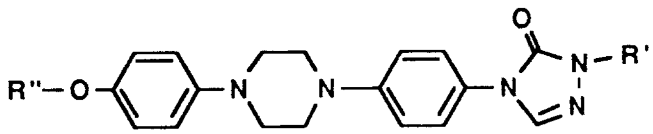

IV

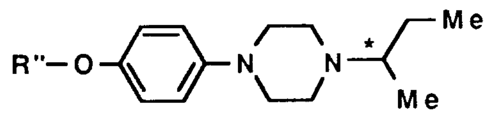

V

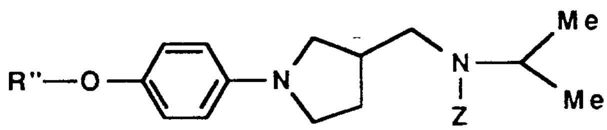

wherein X is independently both F or both Cl or one X is independently F and the other is independently Cl;

R' = $C(_1 - C_{10})$alkyl; $(C_2 - C_{10})$alkenyl; $(C_2 - C_{10})$alkynyl; $(C_3 - C_8)$cycloalky or $CH_2R^2$;
$R^2$ = $(C_1 - C_3)$perhaloalkyl; $CO_2R^3$ $^*-CH(OR^4)CH_2OR^4$ or $CH_2N(R^5)$
$R^3$ = lower alkyl or H
$R^4$ = $R^3$ or $(CH_2)_2OR^3$
$R^5$ = lower alkyl

L is OH or LG; LG is a leaving group;
R" = lower alkyl or Z and
Z = H. or $(C_1 - C_5)$ alkanoyl and the carbons with the asterisks (*) have the R or S absolute configuration

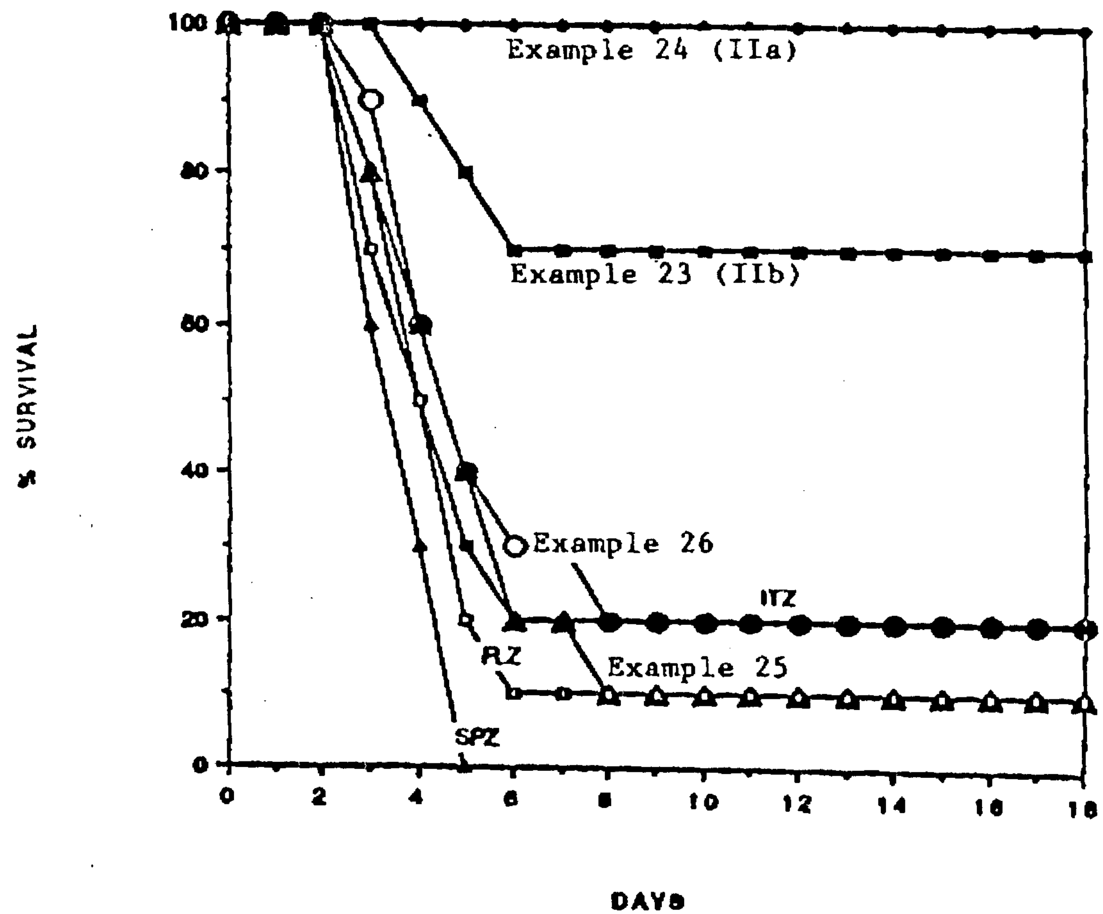


Example 24 - Compound of Formula IIa

Example 23 - Compound of Formula IIb

FLZ - Fluconazole

ITZ - Itraconazole

SPZ - Saperconazole

FIGURE I

European Patent Office

# PARTIAL EUROPEAN SEARCH REPORT

which under Rule 45 of the European Patent Convention shall be considered, for the purposes of subsequent proceedings, as the European search report

Application Number
EP 92 11 8413


## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| D,X | WO-A-8 904 829 (SCHERING CORPORATION)<br>* claims *<br>--- | 1-10 | C07D405/06<br>C07D249/12<br>C07D295/08<br>C07D207/09<br>C07D405/14<br>A61K31/41 |
| D,X | US-A-5 039 676 (A.K. SAKSENA ET AL.)<br>* the whole document *<br>--- | 1-10 | |
| X | EP-A-0 331 232 (JANSSEN PHARMACEUTICA N.V.)<br>*pages25-27,table 7; claims*<br>--- | 10 | |
| Y | EP-A-0 402 989 (JANSSEN PHARMACEUTICA N.V.)<br>* claims *<br>--- | 1-10 | |
| Y | EP-A-0 283 992 (JANSSEN PHARMACEUTICA N.V.)<br>* claims *<br>--- | 1-10 | |
| Y | EP-A-0 173 258 (HOFFMANN-LA ROCHE & CO.)<br>* claims *<br>---<br>-/-- | 1-10 | |

TECHNICAL FIELDS SEARCHED (Int. Cl. 5)

C07D
A61K

## INCOMPLETE SEARCH

The Search Division considers that the present European patent application does not comply with the provisions of the European Patent Convention to such an extent that it is not possible to carry out a meaningful search into the state of the art on the basis of some of the claims
Claims searched completely :
Claims searched incompletely :
Claims not searched :
Reason for the limitation of the search:

see sheet C

| Place of search | Date of completion of the search | Examiner |
|---|---|---|
| THE HAGUE | 15 JANUARY 1993 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

European Patent Office

# PARTIAL EUROPEAN SEARCH REPORT

Application Number

EP 92 11 8413
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| Y | EP-A-0 118 138 (JANSSEN PHARMACEUTICA N.V.)<br>* claims * | 1-10 | |
| Y | EP-A-0 006 711 (JANSSEN PHARMACEUTICA N.V.)<br>* claims * | 1-10 | |
| | | | TECHNICAL FIELDS SEARCHED (Int. Cl. 5) |

EPO FORM 1503 03.82 (P04E10)

Remark: Although claim 9 is directed to a method of treatment of (diagnostic method practised on) the human/animal body (Article 52(4) EPC) the search has been carried out and based on the alleged effects of the compound/composition.